(19) Europäisches Patentamt / European Patent Office / Office européen des brevets

(11) **EP 3 432 890 B1**

(12) **EUROPÄISCHE PATENTSCHRIFT**

(45) Veröffentlichungstag und Bekanntmachung des Hinweises auf die Patenterteilung:
**12.07.2023   Patentblatt 2023/28**

(21) Anmeldenummer: **17713236.2**

(22) Anmeldetag: **22.03.2017**

(51) Internationale Patentklassifikation (IPC):
**A61K 31/59** (2006.01)  **A23K 20/174** (2016.01)
**A23L 33/155** (2016.01)  **A61P 19/08** (2006.01)
**A61P 15/08** (2006.01)  **A23L 33/105** (2016.01)
**A61K 31/593** (2006.01)  **A61K 36/81** (2006.01)
**A61P 25/28** (2006.01)  **A61P 3/10** (2006.01)
**A61P 31/00** (2006.01)  **A61P 39/00** (2006.01)

(52) Gemeinsame Patentklassifikation (CPC):
**A61K 31/59; A23K 20/174; A23L 33/105;
A23L 33/155; A61K 31/593; A61K 36/81;
A61P 3/06; A61P 3/10; A61P 15/08; A61P 19/08;
A61P 25/28; A61P 29/00; A61P 31/00; A61P 39/00**

(86) Internationale Anmeldenummer:
**PCT/EP2017/056871**

(87) Internationale Veröffentlichungsnummer:
**WO 2017/162770 (28.09.2017 Gazette 2017/39)**

(54) **ERZEUGNIS, INSBESONDERE NAHRUNGSMITTELZUSATZ, FUTTERMITTEL, NAHRUNGSMITTEL, PHARMAZEUTIKUM, KOSMETIKUM UND/ODER TIERFUTTERZUSATZ, ZUR ZUFUHR ZUMINDES EINES AN EINEN VITAMIN-D-REZEPTOR BINDENDEN MOLEKÜLS, UND VERFAHREN ZU DESSEN HERSTELLUNG**

PRODUCT, IN PARTICULAR NUTRITIONAL SUPPLEMENT, ANIMAL FEED, FOODSTUFF, DRUG, COSMETIC AND/OR ANIMAL FEED SUPPLEMENT FOR SUPPLYING AT LEAST ONE MOLECULE THAT BONDS TO A VITAMIN D RECEPTOR, AND METHOD FOR THE PRODUCTION THEREOF

PRODUIT, NOTAMMENT COMPLÉMENT ALIMENTAIRE, ALIMENT POUR ANIMAUX, PRODUIT ALIMENTAIRE, PRODUIT PHARMACEUTIQUE, PRODUIT COSMÉTIQUE ET/OU ADDITIF D'ALIMENT POUR ANIMAUX, DESTINÉ À L'APPORT D'AU MOINS UNE MOLÉCULE SE LIANT AU RÉCEPTEUR DE LA VITAMINE D, ET PROCÉDÉ DE FABRICATION ASSOCIÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.03.2016   DE 102016105369**

(43) Veröffentlichungstag der Anmeldung:
**30.01.2019   Patentblatt 2019/05**

(73) Patentinhaber: **Büter, Bernd
8592 Uttwil (CH)**

(72) Erfinder:
• **BERGER BÜTER, Karin
8592 Uttwil (CH)**

• **BÜTER, Bernd
8592 Uttwil (CH)**

(74) Vertreter: **Daub, Thomas
Patent- und Rechtsanwaltskanzlei Daub
Bahnhofstrasse 5
88662 Überlingen (DE)**

(56) Entgegenhaltungen:
**WO-A1-2007/066355     US-A- 5 776 461**

- SAJELI BEGUM AND MADHUR GOYAL A: "Phcog Mag.: Review Article Research and Medicinal Potential of the genus Cestrum (Solanaceae) ? A Review", PHARMACOGNOSY REV, PHARMACOGNOSY NETWORK WORLDWIDE, INDIA, Bd. 1, Nr. 2, Juli 2007 (2007-07), Seiten 320-332, XP007907218, ISSN: 0973-7847
- S. CHENNAIAH ET AL: "Cestrum diurnum leaf as a source of 1,25(OH)2 Vitamin D3 improves egg shell thickness", JOURNAL OF STEROID BIOCHEMISTRY AND MOLECULAR BIOLOGY., Bd. 89-90, Mai 2004 (2004-05), Seiten 589-594, XP055378025, GB ISSN: 0960-0760, DOI: 10.1016/j.jsbmb.2004.03.101
- KOSHY K T: "Vitamin D: an update", JOURNAL OF PHARMACEUTICAL SCIE, AMERICAN PHARMACEUTICAL ASSOCIATION, WASHINGTON, US, Bd. 71, Nr. 2, Februar 1982 (1982-02), Seiten 137-153, XP009147023, ISSN: 0022-3549, DOI: 10.1002/JPS.2600710203
- JESSE F GREGORY III: "Nutritional properties and significance of vitamin glycosides", ANNUAL REVIEW OF NUTRIT, ANNUAL REVIEWS INC., PALO ALTO, CA, US, Bd. 18, Juli 1998 (1998-07), Seiten 277-296, XP008092294, ISSN: 0199-9885, DOI: 10.1146/ANNUREV.NUTR.18.1.277
- PREMA T P ET AL: "Free vitamin D"3 metabolites in Cestrum diurnum leaves", PHYTOCHEMISTRY,, vol. 37, no. 3, 14 November 1994 (1994-11-14), pages 677-681, XP026617152, ISSN: 0031-9422, DOI: 10.1016/S0031-9422(00)90337-6 [retrieved on 1994-11-14]

**Beschreibung**

**Stand der Technik**

Die Erfindung betrifft ein Erzeugnis, insbesondere ein Nahrungsmittelzusatz, ein Futtermittel, ein Nahrungsmittel, ein Pharmazeutikum, ein Kosmetikum und/oder einen Tierfutterzusatz zur Zufuhr zumindest eines an einen Vitamin-D-Rezeptor bindenden Moleküls.

Es ist bekannt, dass die Calcitriol-Derivate Cholecalciferol (von hier an Vitamin D$_3$ genannt), 25-Hydroxy-Vitamin D (von hier an Calcidiol genannt) und 1,25-Dihydroxy-Vitamin D$_3$ (von hier an Calcitriol genannt) zur Behandlung und Prävention von Erkrankungen im Zusammenhang mit Knochenmasse-Verlusten, Knochenfehlentwicklungen, Entzündungen, Infektionskrankheiten, neurodegenerativen Erkrankungen, Strahlenschäden, Diabetes, verminderter Fruchtbarkeit und/oder Hypercholesterinämie bei Tieren (wie beispielsweise bei Haus- oder Nutztiere) und/oder Menschen geeignet ist. Auch eine Anwendung bei Sportlern und/oder körperlich schwer arbeitenden Menschen ist denkbar. Calcitriol-Derivate können beispielsweise aus Pflanzen gewonnen werden. Insbesondere ist bekannt, dass in Pflanzen der Art Cestrum diurnum vergleichsweise große Mengen der oben genannten Verbindungen vorkommen. Oftmals liegen die oben genannten Verbindungen natürlich vorkommend in einer glykosidischen Form vor, so auch im Fall von Cestrum diurnum. Auch synthetische Herstellungsverfahren liefern glykosidische Formen der Verbindungen. Es besteht jedoch ein wissenschaftlicher Konsens, wonach freie Formen der Verbindungen bei einer therapeutischen Anwendung aufgrund einer verbesserten physiologischen Verfügbarkeit eine stärkere und schnellere Wirkung zeigen als die glykosidischen Formen. Mittels üblicher Analytikmethoden wie beispielsweise Chromatographie können dabei lediglich freie Formen von Calcitriol-Derivaten nachgewiesen werden. Glykosidische Formen werden deshalb typischerweise mittels Zugabe von Pansensaft in freie Formen umgewandelt. Eine solche Umwandlung läuft jedoch unvollständig ab, weshalb eine absolute Menge an Calcitriol-Derivaten einer glykosidischen Form in einer Probe nicht bestimmbar und somit eine Quantifizierung auf diese Weise nicht möglich ist. Insbesondere zieht die Verwendung von Pansensaft Probleme hinsichtlich einer Standardisierung und einer Reproduzierbarkeit nach sich, da Pansensaft keine definierte und/oder fixierte Zusammensetzung aufweist.

Der Artikel "BEGUM, A. S. und GOYAL, M. (2007): Phcog Mag - Review Article Research and Medicinal potential of the genus Cestrum (Solanaceae)- A review, erschienen in Pharmacognosy Reviews, Volume 1, Issue 2, Seiten 320 bis 332" offenbart ein Erzeugnis nach dem Oberbegriff des Patentanspruchs 1.

Zudem ist aus der WO 2007/066355 A1 ein Erzeugnis nach dem Oberbegriff des Patentanspruchs 1 bekannt.

Ferner ist bekannt, dass eine therapeutische Anwendung von Hydroxyzimtsäuren den Knochenmetabolismus von Tieren und Pflanzen positiv beeinflusst sowie entzündungshemmende Effekte nach sich ziehen kann. Außerdem können Hydroxyzimtsäuren als Anti-Oxidant wirken und leberstärkende, nervenstärkende, krebsabwehrende, antidiabetische, cholesterinspiegelsenkende und strahlungsschützende Effekte zeigen.

Die Aufgabe der Erfindung besteht insbesondere darin, ein gattungsgemäßes Erzeugnis mit verbesserten Eigenschaften hinsichtlich einer Wirksamkeit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

**Vorteile der Erfindung**

Die Erfindung betrifft ein Erzeugnis, insbesondere einen Nahrungsmittelzusatz, ein Futtermittel, ein Nahrungsmittel, ein Pharmazeutikum, ein Kosmetikum und/oder einen Tierfutterzusatz, zur Zufuhr zumindest eines an einen Vitamin-D-Rezeptor bindenden Moleküls, und zwar Vitamin D$_3$ und/oder Calcitriol und/oder Calcidiol. Das Erzeugnis kann auch ein Nahrungsmittel und/oder ein Pharmazeutikum und/oder ein Kosmetikum oder dergleichen umfassen.

Es wird vorgeschlagen, dass das Erzeugnis zumindest ein biologisches Material mit dem Molekül aufweist.

Hierdurch kann insbesondere eine hohe Wirksamkeit erzielt werden. Vorteilhaft kann ein kostengünstiges und/oder einfach und/oder schnell herstellbares Produkt zur Behandlung von und/oder zur Prävention bei Menschen und/oder Tieren und/oder zur Leistungssteigerung, insbesondere von Zuchttieren, insbesondere Nutztieren, bereitgestellt werden. Ferner können vorteilhafte Eigenschaften hinsichtlich einer physiologischen Verfügbarkeit und/oder einer schnellen Bereitstellung von Vitamin-D-artigen Substanzen erzielt werden.

Vorzugsweise weist das Erzeugnis zumindest ein Trockenpulver und/oder ein Schnittgut und/oder ein Schreddergut auf, welches das biologische Material aufweist. Insbesondere ist denkbar, dass das Erzeugnis das Tockenpulver und/oder das Schnittgut und/oder das Schreddergut in unvergärter oder in zumindest teilweise vergärter Form aufweist. Alternativ oder zusätzlich ist es auch denkbar, dass das Erzeugnis zumindest einen Extrakt, insbesondere einen Trockenextrakt oder einen Flüssigextrakt, aufweist, welcher das biologische Material aufweist.

In einer nicht durch die beanspruchte Erfindung abgedeckten Ausführungsform ist außerdem ein Verfahren denkbar zur Bestimmung eines quantitativen Mengenverhältnisses von an einen Vitamin-D-Rezeptor bindenden Molekülen, insbesondere Biomolekülen, insbesondere von Calcitriol-Derivaten, die in zumindest einer freien Form und in zumindest einer glykosidischen Form in einem biologischen, insbesondere einem pflanzlichen Material vorliegen, wobei in zumindest einem Vorbehandlungsschritt eine Umwandlung von Molekülen der glykosidischen Form in Moleküle der freien Form durchgeführt wird. Die im Folgenden beschriebenen Verfahren sind nicht Teil der beanspruchten Erfindung.

Unter einer "Bestimmung eines quantitativen Mengenverhältnisses" zweier oder mehrerer Substanzen soll insbesondere eine Datenerfassung und/oder Messung und/oder Datenauswertung verstanden werden, welche als Ergebnis zumindest ein Verhältnis an Mengen, beispielsweise an Stoffmengen und/oder an Masseanteilen und/oder an Volumina der Substanzen liefert, welches um weniger als einen Faktor 5, vorteilhaft um weniger als einen Faktor 2, besonders vorteilhaft um weniger als einen Faktor 1,5, vorzugsweise um weniger als einen Faktor 1,2 und besonders bevorzugt um weniger als einen Faktor 1,1 von dem wahren, insbesondere paarweisen, Verhältnis an Mengen abweicht. Unter "an einen Vitamin-D-Rezeptor bindenden Molekülen" sollen insbesondere Moleküle verstanden werden, die unter Normalbedingungen, insbesondere in wässriger Lösung, mit einem Vitamin-D-Rezeptor, insbesondere mit einem Vitamin-$D_3$-Rezeptor, einen ECso-Wert (50% effective concentration) von wenigstens 100 pM, vorteilhaft von wenigstens 200 pM und besonders vorteilhaft von wenigstens 500 pM und/oder einen ECso-Wert von höchstens 100 nM, vorteilhaft von höchstens 10 nM und besonders vorteilhaft von höchstens 1 nM zeigen. Insbesondere können an einen Vitamin-D-Rezeptor bindende Moleküle eine Aktivierung eines Vitamin-D-Rezeptors hervorrufen. Vorzugsweise wird zum Nachweis solcher Moleküle zumindest ein Bioassay mit einem entsprechenden Vitamin-D-Rezeptor, beispielsweise einem Vitamin-$D_3$-Rezeptor, durchgeführt. Insbesondere wird in dem Bioassay eine Dissoziationskonstante bestimmt. Unter einem Derivat einer Ausgangsverbindung soll insbesondere ein abgeleiteter Stoff verstanden werden, welcher eine ähnliche chemische Struktur wie die Ausgangsverbindung aufweist, wobei insbesondere zumindest ein Wasserstoffatom der Ausgangsverbindung in dem Derivat durch zumindest eine funktionelle Gruppe und/oder zumindest eine funktionelle Gruppe der Ausgangsverbindung in dem Derivat durch zumindest ein Wasserstoffatom und/oder zumindest eine funktionelle Gruppe der Ausgangsverbindung in dem Derivat durch zumindest eine andere funktionelle Gruppe ersetzt ist/sind. Vorteilhaft weist ein Derivat relativ zu einer Ausgangsverbindung höchstens zehn, besonders vorteilhaft höchstens sieben, vorzugsweise höchstens fünf und besonders bevorzugt höchstens drei derartige Ersetzungen auf. Insbesondere kann ein Derivat einer Verbindung ein Ester der Verbindung sein. Bekannte Calcitriol-Derivate sind insbesondere Calcitriol, Calcidiol und Vitamin $D_3$. Das Verfahren soll aber insbesondere nicht auf diese Calcitriol-Derivate eingeschränkt sein.

Unter einer "glykosidischen Form" eines Moleküls soll insbesondere eine Verbindung verstanden werden, die ausgehend von dem Molekül mittels zumindest einer Glykosylierung erzeugbar ist. Insbesondere ist die Verbindung ausgehend von dem Molekül lediglich mittels einer oder mehrerer Glykosylierungen erzeugbar. Vorteilhaft ist ausgehend von dem Molekül die Verbindung mittels höchstens zehn, besonders vorteilhaft höchstens fünf, bevorzugt höchstens drei und besonders bevorzugt höchstens zwei Glykosylierungen erzeugbar. Unter einer freien Form eines Moleküls soll insbesondere eine von einer glykosidischen Form verschiedene Form des Moleküls verstanden werden. Vorzugsweise ist die freie Form eines Moleküls mittels lediglich zumindest einer Glykosylierung in eine glykosidische Form umwandelbar.

Unter einem biologischen Material soll insbesondere ein Material verstanden werden, das genetische Informationen enthält und sich selbst reproduzieren oder in einem biologischen System reproduziert werden kann und/oder das mittels insbesondere von biologischen und/oder chemischen Methoden und von verschiedenen physikalischen Methoden aus einem Ausgangsmaterial gewonnen werden kann, welches genetische Informationen enthält und sich selbst reproduzieren oder in einem biologischen System reproduziert werden kann. Es handelt sich bei dem biologischen Material um, insbesondere getrocknete, Pflanzen und/oder Pflanzenteile und/oder zumindest einen daraus gewonnenen Extrakt und/oder eine daraus hergestellte Lösung und/oder Dispersion.

Vorteilhaft wird von einer Gesamtprobe eines biologischen Materials eine Referenzprobe für den Vorbehandlungsschritt entnommen, insbesondere eine Referenzprobe mit einem definierten Volumen und/oder einer definierten Masse. Bei der Gesamtprobe kann es sich beispielsweise um ein Trockenpulver handeln. Besonders vorteilhaft wird die Referenzprobe in zumindest einem, vorteilhaft hydrophilen, Lösungsmittel, vorzugsweise in Wasser, aufgenommen. Es ist aber auch denkbar, dass die Gesamtprobe zumindest ein Lösungsmittel, vorzugsweise Wasser, enthält und insbesondere die Messprobe direkt entnommen wird. Insbesondere werden Moleküle der glykosidischen Form mittels Wasser, insbesondere mit zumindest einem zusätzlichen Antioxidationsmittel wie beispielsweise Zitronensäure und/oder zumindest ein Gallat und/oder zumindest ein Sulfit und/oder Milchsäure und/oder Ascorbinsäure und/oder zumindest einem Ascorbat-Salz und/oder Zink und/oder Selen und/oder zumindest ein Diphosphat und/oder Zinnchlorid extrahiert. Vorteilhaft wird zumindest ein Flüssigextrakt mit Molekülen der glykosidischen Form durch Schütteln und/oder Rühren und/oder Behandlung in zumindest einem Ultraschallbad, insbesondere bei einer Temperatur zwischen 20°C und 50°C, beispielsweise bei einer Temperatur von 25°C oder von 30°C oder von 40°C, extrahiert. Dabei ist insbesondere denkbar, verschiedene Schritte bei verschiedenen Temperaturen und/oder wiederholt und/oder in wechselnder Abfolge durchzuführen. Vorzugsweise enthält der Flüssigextrakt, der aus der Referenzprobe gewonnen wird, höchstens Spuren von Molekülen der freien Form.

Insbesondere werden während des Vorbehandlungsschritts wenigstens 20 %, vorteilhaft wenigstens 50 %, besonders vorteilhaft wenigstens 80 %, vorzugsweise wenigstens 90 % und besonders bevorzugt wenigstens 95 % der Moleküle der glykosidischen Form, insbesondere der Referenzprobe, in Moleküle der freien Form umgewandelt. Es ist denkbar, dass das biologische Material verschiedene glykosidische Formen sowie eine freie Form von Molekülen enthält. In diesem Fall ist vorteilhaft denkbar, dass für jede glykosidische Form ein jeweils angepasster Vorbehandlungsschritt durchgeführt wird, welcher jeweils lediglich einige, vorzugsweise genau eine, der glykosidischen Formen in die freie Form umwandelt, insbesondere jedoch nicht die von den entsprechenden glykosidischen Formen bzw. von der entsprechenden glykosidischen Form verschiedenen glykosidischen Formen. Vorteilhaft wird die Umwandlung in einem Lösungsmittel durchgeführt, besonders vorteilhaft in dem Lösungsmittel des Flüssigextrakts. Insbesondere werden Moleküle der freien Form nach der Umwandlung mittels zumindest eines, insbesondere organischen, vorteilhaft lipophilen, Lösungsmittels, vorzugsweise mittels Methanol und/oder Ethanol und/oder Ethylacetat und/oder Dichlormethan und/oder Hexan und/oder Tetrahydrofuran (THF), extrahiert. Vorzugsweise wird anschließend das organische Lösungsmittel abgeschieden und insbesondere unter Vakuum verdampft, um einen Trockenextrakt zu erhalten, welcher insbesondere Moleküle der freien Form aufweist, deren Vorkommen vorteilhaft zumindest teilweise auf die Umwandlung zurückzuführen ist. Vorzugsweise wird der Trockenextrakt in einem Lösungsmittel oder einem Lösungsmittelgemisch aufgenommen. Besonders bevorzugt ist das Lösungmittel oder das Lösungsmittelgemisch an eine mobile Phase eines Hochleistungsflüssigkeitschromatographen angepasst.

Durch ein derartiges nicht erfindungsgemäßes Verfahren kann vorteilhaft eine Quantifizierbarkeit erzielt werden. Vorteilhaft kann ein Mengenverhältnis genau bestimmt werden. Ferner kann vorteilhaft ein Mengenverhältnis bestimmt werden, dass nahe an einem wahren Mengenverhältnis liegt. Vorteilhaft kann ein Absolutgehalt an Zielmolekülen genau bestimmt werden. Weiterhin wird vorteilhaft eine schnelle und zuverlässige Messung ermöglicht. Außerdem wird vorteilhaft ein kostengünstig und/oder reproduzierbar durchführbares Analyseverfahren bereitgestellt. Insbesondere ist das Verfahren vorteilhaft in einem Labormaßstab und in einem großtechnischen Maßstab durchführbar. Ferner kann ein biologisches Material insbesondere im Hinblick auf eine Anwendbarkeit in Nahrungsergänzungsmitteln, Tierfuttern und/oder Tierfutterzusätzen untersucht werden. Insbesondere kann ein biologisches Material vorteilhaft schnell und/oder zuverlässig auf eine Tauglichkeit als Quelle für Vitamin-D und/oder Vitamin-D-ähnliche Substanzen untersucht werden. Vorteilhaft kann eine potentielle Wirksamkeit eines biologischen Materials ermittelt werden. Ferner können vorteilhaft für eine Züchtung geeignete Organismen zuverlässig ermittelt werden. Insbesondere kann eine Eignung eines Organismus als potentieller Lieferant freier Calcitriol-Derivate zuverlässig erkannt werden. Ferner kann vorteilhaft ein schnelles und/oder zuverlässiges Screening potentieller Quellen freier Calcitriol-Derivate ermöglicht werden.

In einer weiteren nicht erfindungsgemäßen Ausführungsform wäre denkbar, dass die Umwandlung enzymatisch durchgeführt wird. Insbesondere wird in dem Vorbehandlungsschritt zumindest ein Enzym zu der Messprobe zugegeben. Vorzugsweise wird in dem Vorbehandlungsschritt zumindest ein Enzymgemisch zu der Messprobe zugegeben, welches zumindest zwei verschiedene Enzyme enthält. Hierdurch kann vorteilhaft eine hohe Effizienz einer Umwandlung von Molekülen der glykosidischen Form in Moleküle der freien Form ermöglicht werden.

In einer weiteren nicht erfindungsgemäßen Ausgestaltung wäre denkbar, dass die Umwandlung mittels eines Enzymgemischs durchgeführt wird, welches zumindest zwei unterschiedliche Hydrolasen aufweist. Hierdurch kann vorteilhaft eine hohe Reproduzierbarkeit und/oder Kosteneffizienz erzielt werden. Vorteilhaft ist wenigstens eine der Hydrolasen und besonders vorteilhaft sind alle verwendeten Hydrolasen pflanzlichen, mikrobiellen, tierischen und/oder fungalen Ursprungs, wodurch insbesondere niedrige Kosten erzielbar sind. Insbesondere sind die Hydrolasen Enzyme, die ein Aufbrechen glykosidischer Bindungen bewirken. Bei den Hydrolasen kann es sich beispielsweise jeweils um eine Amylase, eine Glucanase, eine Cellulase, eine Inulinase, eine Xylanase, eine Galactosaminase, eine Dextranase, eine Pektinase, eine Galactosidase, eine Glucosidase, eine Mannosidase, eine Fructofuranosidase, eine Fucosidase, eine Rhamnosidase, eine Arabinofuranosidase, eine Maltotetraohydrolase, eine Levanase, eine Licheninase, eine Fructosidase, eine Agarase, eine Carrageenase, eine Cellobiosidase, eine Chitinase, eine Galacturonase, eine Fructofuranosidase oder dergleichen handeln. Vorteilhaft enthält das Enzymgemisch zumindest eine Glucosidase und zumindest eine weitere Hydrolase, insbesondere zumindest eine weitere der genannten Hydrolasen. Es ist denkbar, dass das Enzymgemisch wenigstens drei oder wenigstens vier oder wenigstens fünf oder noch mehr unterschiedliche Hydrolasen umfasst, wodurch insbesondere eine präzise Anpassung des Vorbehandlungsschritts an unterschiedliche Bedingungen erzielt werden kann.

In einer weiteren nicht durch die beanspruchte Erfindung abgedeckten Ausführungsform ist denkbar, dass in zumindest einem Messschritt ein Messgehalt an Molekülen der freien Form bestimmt wird. Vorteilhaft wird in dem Messschritt von der Gesamtprobe des biologischen Materials eine Messprobe, insbesondere vor dem Vorbehandlungsschritt, entnommen, insbesondere eine Messprobe mit einem definierten Volumen und/oder einer definierten Masse. Besonders vorteilhaft wird die Messprobe in zumindest einem Lösungsmittel, vorzugsweise Methanol und/oder Ethanol und/oder Ethylacetat und/oder Dichlormethan und/oder Hexan und/oder THF, gelöst und/oder aufgenommen. Es ist aber auch denkbar, dass die Gesamtprobe zumindest ein Lösungsmittel, vorzugsweise Methanol und/oder Ethanol und/oder Ethylacetat und/oder Dichlormethan und/oder Hexan und/oder THF, enthält und insbesondere die Messprobe direkt entnommen wird. Insbesondere werden Moleküle der freien Form mittels zumindest eines, insbesondere organischen, vorteilhaft lipophilen, Lösungsmittels wie beispielsweise Methanol und/oder Ethanol und/oder Ethylacetat und/oder Dichlormethan und/oder Hexan und/oder THF extrahiert. Vorteilhaft wird zumindest ein Flüssigextrakt mit Molekülen der freien Form durch Schütteln und/oder Rühren und/oder Behandlung in zumindest einem Ultraschallbad, insbesondere bei einer Temperatur zwischen 20°C und 50°C, beispielsweise bei einer Temperatur von 25°C oder von 30°C oder von 40°C, extrahiert. Dabei ist insbesondere denkbar, verschiedene Schritte bei verschiedenen Temperaturen und/oder wiederholt und/oder in wechselnder Abfolge durchzuführen. Vorzugsweise wird der Flüssigextrakt zumindest einmal gefiltert, um ein Filtrat zu erhalten. Es ist auch denkbar, zumindest eine Zentrifugation des Flüssigextrakts durchzuführen. Vorteilhaft enthält der Flüssigextrakt, welcher aus der Messprobe gewonnen wird, höchstens Spuren von Molekülen der glykosidischen Form. Vorzugsweise wird in einem weiteren Schritt Lösungsmittel des Flüssigextrakts, vorzugsweise des Filtrats, verdampft, um einen Trockenextrakt zu erhalten. Vorzugsweise wird der Trockenextrakt in einem Lösungsmittel oder einem Lösungsmittelgemisch aufgenommen. Besonders bevorzugt ist das Lösungsmittelgemisch an eine mobile Phase eines Hochleistungsflüssigkeitschromatographen angepasst. Vorteilhaft wird in dem Messschritt ein Gehalt an Molekülen der freien Form bestimmt. Besonders vorteilhaft wird in dem Messschritt der Messgehalt aus dem Gehalt an Molekülen der freien Form, insbesondere in der Messprobe, ermittelt und/oder wird der Messgehalt von dem Gehalt an Molekülen der freien Form, insbesondere in der Messprobe, gebildet. Insbesondere entspricht der Messgehalt einem Gehalt an Molekülen der freien Form in dem biologischen Material. Hierdurch kann vorteilhaft ein einfaches und schnelles analytisches Verfahren bereitgestellt werden. Ferner kann hierdurch eine hohe Reproduzierbarkeit und/oder eine hohe Genauigkeit erzielt werden.

In einer weiteren nicht erfindungsgemäßen Ausführungsform ist zudem denkbar, dass in zumindest einem Referenzbildungsschritt ein Referenzgehalt an Molekülen der freien Form nach dem Vorbehandlungsschritt bestimmt wird. Insbesondere wird in dem Referenzbildungsschritt ein Gesamtgehalt an freien Molekülen in der Referenzprobe nach dem Vorbehandlungsschritt bestimmt. Vorzugsweise entspricht der Referenzgehalt einem Gehalt an Molekülen der glykosidischen Form vor dem Vorbehandlungsschritt, in welchem bevorzugt, insbesondere wie oben erwähnt, Moleküle der glykosidischen Form in Moleküle der freien Form umgewandelt werden. Besonders vorteilhaft entspricht der Referenzgehalt einem Gehalt an Molekülen der glykosidischen Form in dem biologischen Material. Ein Gesamtgehalt an Molekülen der freien Form und der glykosidischen Form in dem biologischen Material entspricht vorteilhaft einer Summe des Messgehalts und des Referenzgehalts. Vorzugsweise kann das Mengenverhältnis aus dem Referenzgehalt und dem Messgehalt bestimmt werden, indem ein Quotient aus dem Messgehalt und dem Referenzgehalt gebildet wird. Das derart bestimmte Mengenverhältnis entspricht vorzugsweise einem Verhältnis des Gehalts an Molekülen der freien Form und des Gehalts an Molekülen der glykosidischen Form in dem biologischen Material. Hierdurch kann vorteilhaft eine genaue Messung und/oder eine hohe Reproduzierbarkeit erzielt werden. Ferner können vorteilhaft Konzentrationen verschiedener Verbindungen in separaten Schritten bestimmt werden, wodurch insbesondere Messungenauigkeiten reduziert und/oder Messfehler vermieden werden können.

In einer nicht erfindungsgemäßen Ausgestaltung könnte das Mengenverhältnis mittels Hochleistungsflüssigkeitschromatographie (HPLC) bestimmt werden. Insbesondere wird ein Gehalt an Molekülen der freien Form während des Messschritts und/oder während des Vorbehandlungsschritts und/oder während des Referenzbildungsschritts mittels HPLC bestimmt. Vorzugsweise wird eine Detektion von Molekülen der freien Form im UV-Bereich, besonders bevorzugt bei einer Wellenlänge von 265 nm, durchgeführt. Vorzugsweise wird Calcitriol als Referenz für eine Kalibration und/oder eine Justierung verwendet. Hierdurch kann vorteilhaft eine hohe Zuverlässigkeit und/oder eine hohe Messgenauigkeit und/oder eine kostengünstig und/oder schnell durchführbare Analytikmethode bereitgestellt werden.

Zudem ist in einer nicht erfindungsgemäßen Ausführungsform denkbar, dass in zumindest einem Extraktionsschritt zumindest ein Teil der Moleküle der glykosidischen Form mittels zumindest eines Extraktionsmittels, insbesondere eines Lösungsmittels und/oder eines Lösungsmittelgemischs, aus dem biologischen Material extrahiert wird, das weniger polar ist als Wasser. Vorzugsweise wird in dem Extraktionsschritt zumindest einmal mit Wasser und zumindest einmal mit dem Extraktionsmittel extrahiert, insbesondere aus unterschiedlichen Messproben oder konsekutiv aus einer einzelnen Messprobe, wobei vorteilhaft zunächst mit Wasser und anschließend mittels des Lösungsmittels extrahiert wird, jedoch auch der umgekehrte Fall grundsätzlich denkbar ist. Das Extraktionsmittel kann ein einzelnes Lösungsmittel sein. Vorteilhaft ist das Extraktionsmittel ein Lösungsmittelgemisch. Besonders bevorzugt weist das Extraktionsmittel Wasser und zumindest ein weiteres, von Wasser verschiedenes Lösungsmittel auf, wobei das von Wasser verschiedene Lösungsmittel eine geringere Polarität als Wasser aufweist. Beispielsweise kann das Extraktionsmittel, insbesondere zusätzlich zu Wasser, Methanol und/oder Ethanol und/oder Isopropanol und/oder Pyridin und/oder Acetonitril und/oder Dimethylsulfoxid und/oder Ethylacetat und/oder Tert-Butylmethyether und/oder Tetrahydrofuran und/oder Dioxan und/oder Aceton enthalten. Vorzugsweise enthält das Extraktionsmittel wenigstens 10 %, vorteilhaft wenigstens 20 % und besonders vorteilhaft wenigstens 30 % Wasser. Bevorzugt enthält das Extraktionsmittel höchstens 90 %, vorteilhaft höchstens 80 % und besonders vorteilhaft höchstens 70 % Wasser. Vorzugsweise enthält das Extraktionsmittel Wasser und ein weiteres Lösungsmittel, besonders bevorzugt in einem Mischungsverhältnis von etwa 3:7 oder 2:3 oder 1:1, insbesondere bezogen auf ein Volumen. Insbesondere werden die in dem Extraktionsschritt entnommenen Moleküle analog zu dem Referenzbildungsschritt in Moleküle der freien Form umgewandelt. Vorzugsweise wird die Konzentration der in dem Extraktionsschritt entnommenen Moleküle anhand einer Konzentration an Molekülen der freien Form, in welche die entnommenen Moleküle insbesondere enzymatisch umgewandelt wurden, ermittelt, insbesondere analog zu dem Fall der Moleküle der glykosidischen Form in dem Referenzbildungsschritt. Vorzugsweise handelt es sich bei den in dem Extraktionsschritt extrahierten Molekülen um einfach glykosidische Moleküle, also insbesondere um eine Teilmenge der Moleküle der glykosidischen Form. Es ist denkbar, den Umwandlungsschritt und/oder den Referenzbildungsschritt nach Entnahme der einfach glykosidischen Moleküle in dem Extraktionsschritt durchzuführen, wobei insbesondere eine Konzentration mehrfach glykosidischer Moleküle vorzugsweise als Referenz ermittelt wird. Hierdurch können vorteilhaft ein Mengenverhältnis von Molekülen der freien Form und von Molekülen der glykosidischen Form, insbesondere einer mehrfach glykosidischen Form und/oder ein Mengenverhältnis von Molekülen einer einfach glykosidischen Form und Molekülen der glykosidischen Form, insbesondere der mehrfach glykosidischen Form bestimmt werden.

In einer weiteren nicht durch die beanspruchte Erfindung abgedeckten Ausführungsform wäre ferner die Verwendung des Verfahrens denkbar zur Bestimmung eines quantitativen Mengenverhältnisses von an einen Vitamin-D-Rezeptor bindenden Molekülen zur Identifizierung eines Organismus, insbesondere einer Pflanze, welcher zumindest ein biologisches Material aufweist, in welchem an einen Vitamin-D-Rezeptor bindende Moleküle, insbesondere Calcitriol-Derivate, in zumindest einer freien Form und in zumindest einer glykosidischen Form, insbesondere in einem bestimmten Mengenverhältnis, vorliegen, wobei an einen Vitamin-D-Rezeptor bindende Moleküle nachgewiesen werden. Insbesondere enthält das biologische Material die Moleküle in einer Konzentration von wenigstens 1 $\mu$g/g, vorteilhaft von wenigstens 10 $\mu$g/g, besonders vorteilhaft von wenigstens 20 $\mu$g/g, vorzugsweise von wenigstens 50 $\mu$g/g und besonders bevorzugt von wenigstens 100 $\mu$g/g. Das Mengenverhältnis kann beispielsweise 1:1000, 1:100, 1:10, 1:5, 1:3, 2:3, 1:2, 1:1, 2:1, 3:2, 3:1, 5:1, 10:1, 100:1, 1000:1 oder in denkbarer Weise anders von diesen Werten sein. Vorteilhaft wird zumindest eine Gesamtprobe aus dem Organismus entnommen, beispielsweise im Fall einer Pflanze, insbesondere eines Makrophyten, oder es wird eine Gesamtprobe gebildet, die den Organismus enthält, beispielsweise im Fall von Bakterien oder im Fall eines Mikrophyten oder im Fall von Algen oder im Fall von Pilzen. Besonders vorteilhaft wird das Mengenverhältnis in der Gesamtprobe bestimmt. Hierdurch kann vorteilhaft eine Quelle für physiologisch aktives Vitamin D und/oder für physiologisch aktive Vitamin-D-ähnliche Substanzen aufgefunden werden. Ferner kann hierdurch vorteilhaft ein schnelles und/oder kostengünstiges und/oder zuverlässigen Screening durchgeführt werden.

Insbesondere handelt es sich bei dem biologischen Material des Erzeugnisses um biologisches Material eines in obiger Verwendung identifizierten Organismus. Hierdurch kann vorteilhaft ein Produkt, insbesondere zur therapeutischen Verwendung in einer Behandlung und/oder Prävention und/oder zur Leistungssteigerung bei Zuchttieren, insbesondere bei Nutztieren, mit einem bestimmten Mengenverhältnis an freien und glykosidischen Vitamin-D-artigen Molekülen bereitgestellt werden.

Das Molekül in dem biologischen Material liegt in zumindest einer ersten Form, zumindest einer zweiten Form und zumindest einer dritten Form vorliegt, wobei die erste Form eine freie Form, die zweite Form eine einfach glykosidische Form und die dritte Form eine mehrfach glykosidische Form ist. Hierdurch kann vorteilhaft eine physiologische Zugänglichkeit verbessert werden. Ferner kann hierdurch eine schnelle und/oder starke und/oder nachhaltige Wirkweise ermöglicht werden.

Zudem wäre in einer nicht erfindungsgemäßen Ausführungsform eine Verwendung des Verfahrens denkbar zur Bestimmung eines quantitativen Mengenverhältnisses von an einen Vitamin-D-Rezeptor bindenden Molekülen zur Identifizierung eines Organismus, insbesondere einer Pflanze, welcher zumindest ein biologisches Material aufweist, in welchem an einen Vitamin-D-Rezeptor bindende Moleküle, insbesondere Calcitriol-Derivate, in zumindest einer ersten Form, zumindest einer zweiten Form und zumindest einer dritten Form vorliegen, wobei die erste Form eine freie Form, die zweite Form eine einfach glykosidische Form und die dritte Form eine mehrfach glykosidische Form ist. Insbesondere enthält das biologische Material die Moleküle in einer Konzentration von wenigstens 50 $\mu$g/g und besonders bevorzugt von wenigstens 100 $\mu$g/g. Ein Mengenverhältnis zwischen Molekülen der ersten Form und Molekülen der dritten Form kann beispielsweise 1:1000, 1:100, 1:10, 1:5, 1:3, 2:3, 1:2, 1:1, 2:1, 3:2, 3:1, 5:1, 10:1, 100:1, 1000:1 oder in denkbarer Weise abweichend von diesen Werten sein. Alternativ oder zusätzlich kann ein Mengenverhältnis zwischen Molekülen der ersten und der zweiten Form (insbesondere folglich einer Summe einer Menge von Molekülen der ersten Form und Molekülen der zweiten Form) und Molekülen der dritten Form beispielsweise 1:1000, 1:100, 1:10, 1:5, 1:3, 2:3, 1:2, 1:1, 2:1, 3:2, 3:1, 5:1, 10:1, 100:1, 1000:1 oder in denkbarer Weise abweichend von diesen Werten sein.

Vorteilhaft wird zumindest eine Gesamtprobe aus dem Organismus entnommen, beispielsweise im Fall einer Pflanze, insbesondere eines Makrophyten, oder es wird eine Gesamtprobe gebildet, die den Organismus enthält, beispielsweise im Fall von Bakterien oder im Fall eines Mikrophyten oder im Fall von Algen oder im Fall von Pilzen. Besonders vorteilhaft wird das Mengenverhältnis in der Gesamtprobe bestimmt. Hierdurch kann vorteilhaft eine Quelle für physiologisch aktives Vitamin D und/oder für physiologisch aktive Vitamin-D-ähnliche Substanzen, insbesondere für Moleküle der ersten Form und/oder der zweiten Form, aufgefunden werden. Ferner kann hierdurch vorteilhaft ein schnelles und/oder kostengünstiges und/oder zuverlässigen Screening durchgeführt werden.

Insbesondere handelt es sich bei dem biologischen Material des Erzeugnisses um biologisches Material eines in obiger Verwendung identifizierten Organismus. Hierdurch kann vorteilhaft ein Produkt, insbesondere zur therapeutischen Verwendung in einer Behandlung und/oder Prävention und/oder zur Leistungssteigerung bei Nutztieren mit einem bestimmten Mengenverhältnis an freien und glykosidischen Vitamin-D-artigen Molekülen bereitgestellt werden.

In einer vorteilhaften Ausgestaltung der Erfindung wird vorgeschlagen, dass ein Mengenverhältnis von Molekülen, die in der ersten oder der zweiten Form vorliegen, und von Molekülen, die in der dritten Form vorliegen, wenigstens 1:1, vorteilhaft wenigstens 3:2 beträgt. Hierdurch kann vorteilhaft eine physiologische Zugänglichkeit verbessert werden. Ferner kann hierdurch eine schnelle und/oder starke und/oder nachhaltige Wirkweise ermöglicht werden.

In einer besonders vorteilhaften Ausgestaltung der Erfindung wird vorgeschlagen, dass das biologische Material zumindest ein von Vitamin $D_3$, 25-Hydroxy-Vitamin D und 1$\alpha$,25-Dihydroxy-Vitamin $D_3$ verschiedenes an einen Vitamin-D-Rezeptor bindendes weiteres Molekül aufweist, insbesondere ein Biomolekül, vorteilhaft ein weiteres Calcitriol-Derivat. Beispielsweise unterscheidet sich das weitere Molekül von Calcitriol durch ein oder zwei zusätzliche und/oder fehlende und/oder ausgetauschte funktionelle Gruppen und/oder Wasserstoffatome. Insbesondere liegt das weitere Molekül in dem biologischen Material in zumindest einer freien Form und in zumindest einer glykosidischen Form vor. Insbesondere beträgt ein Mengenverhältnis von weiteren Molekülen der freien Form und weiteren Molekülen der glykosidischen Form wenigstens 1:4, vorteilhaft wenigstens 3:7, besonders vorteilhaft wenigstens 2:3, vorteilhafter Weise 1:1, vorzugsweise wenigstens 3:2, bevorzugt wenigstens 7:3 und besonders bevorzugt wenigstens 4:1. Vorzugsweise weist das weitere Molekül ein UV-Spektrum auf, welches, insbesondere für Wellenlängen kleiner 300 nm und/oder größer 200 nm, relativ zu einem UV-Spektrum von Calcitriol einen Match Factor von wenigstens 900, vorteilhaft von wenigstens 910, besonders vorteilhaft von wenigstens 920 und bevorzugt von wenigstens 930 zeigt. Identische Peaks zweier Spektren zeigen insbesondere einen Match Factor von 1000. Insbesondere ist der Match Factor für ein erstes Spektrum und ein zweites Spektrum gegeben als

$$Match\ Factor = \frac{10^3 \times \left(\sum x \times y - \left(\frac{\sum x \times \sum y}{n}\right)\right)^2}{\left(\sum x^2 - \left(\frac{\sum x \times \sum x}{n}\right)\right) \times \left(\sum y^2 - \left(\frac{\sum y \times \sum y}{n}\right)\right)},$$

wobei x und y gemessene Absorptionswerte des ersten beziehungsweise des zweiten Spektrums für jeweils eine Wellenlänge sind und n einer Anzahl von Datenpunkten x beziehungsweise y entspricht. Hierdurch kann vorteilhaft eine hohe Wirksamkeit erzielt werden. Ferner kann hierdurch ein Wirkspektrum vorteilhaft erweitert werden.

Das biologische Material enthält zumindest ein Hydroxyzimtsäure-Derivat in einer Konzentration von wenigstens 100 μg/g, vorteilhaft von wenigstens 200 μg/g, besonders vorteilhaft von wenigstens 500 μg/g, vorzugsweise von wenigstens 1000 μg/g und bevorzugt von wenigstens 2000 μg/g und besonders bevorzugt von wenigstens 5000 μg/g. Bei dem Hydroxyzimtsäure-Derivat handelt es sich um 2-Hydroxyzimtsäure, 3-Hydroxyzimtsäure, 4-Hydroxyzimtsäure, 3,4,-Dihydroxyzimtsäure, Chlorogensäure, Cumarin, Ferulasäure oder Sinapinsäure oder es kann sich zusätzlich auch um einen Hydroxyzimtsäure-Ester wie beispielsweise Kaffeesäure-Ester handeln. Insbesondere kann das biologische Material verschiedene Hydroxyzimtsäure-Derivate aufweisen. Hierdurch kann vorteilhaft eine Quelle für eine besonders schnell und/oder zuverlässig und/oder stark wirkende Stoffkombination bereitgestellt werden. Vorteilhaft kann ein synergetischer Effekt durch ein Zusammenspiel zumindest eines, insbesondere freien, Calcitriol-Derivats mit zumindest einem Hydroxyzimtsäure-Derivat erzeugt werden.

In einer vorteilhaften Ausgestaltung der Erfindung wird vorgeschlagen, dass das biologische Material von zumindest einer Pflanze der Gattung Cestrum stammt. Es ist denkbar, dass das biologische Material von Pflanzen verschiedener Art einer gemeinsamen Gattung Cestrum stammt. Beispielsweise kann das Erzeugnis ein Schnittgut und/oder ein Pulver und/oder einen Extrakt, insbesondere einen Flüssigextrakt und/oder einen Trockenextrakt, aufweisen, welche/welches aus Pflanzenteilen der Pflanze wie beispielsweise Blättern und/oder Wurzeln und/oder Blüten und/oder Früchten und/oder zumindest einem Stamm und/oder zumindest einem Stängel und/oder aus der gesamten Pflanze gewonnen wurde/wurden. Es ist insbesondere auch denkbar, dass eine Pflanze mit entsprechendem Nährwert als direkte Quelle für ein Tierfutter dient. Ein als Tierfutter ausgebildetes Erzeugnis weist insbesondere lediglich Pflanzenteile auf. Hierdurch kann insbesondere eine Verwendung einer kostengünstigen und/oder leicht zu kultivierenden Quelle für ein biologisches Material mit gewünschten Inhaltsstoffen ermöglicht werden.

In einer besonders vorteilhaften Ausgestaltung der Erfindung wird vorgeschlagen, dass die Pflanze von der Art Cestrum diurnum ist. Es ist aber auch denkbar, dass die Pflanze von der Art Cestrum nocturnum oder Cestrum parqui ist. Ferner sind beliebige andere Arten der Gattung Cestrum denkbar, wie beispielsweise auch Cestrum aurantiacum oder Cestrum nocturnum x, wobei die Erfindung grundsätzlich alle denkbaren Arten von Cestrum umfasst, die selbstverständlich an dieser Stelle nicht vollständig aufgezählt sein sollen. Hierdurch kann vorteilhaft eine kostengünstige und/oder zuverlässige Quelle für freie Calcitriol-Derivate und/oder Hydroxyzimtsäure-Derivate bereitgestellt werden.

Alternativ ist denkbar, dass es sich bei der Pflanze um eine Pflanze der Gattung Solanum, beispielsweise um Solanum nigrum oder Solanum hispidum oder Solanum verbascifolium, oder Solanum torvum oder Solanum trilobatum oder um eine Pflanze der Gattung Trisetum, beispielsweise um Trisetum flavescence, oder um eine Pflanze der Gattung Nierembergia, beispielsweise um Nierembergia veitchii, oder um eine Pflanze der Gattung Sauropus, beispielsweise Sauropus androgynus handelt.

Das Molekül in dem biologischen Material liegt in zumindest einer ersten Form, zumindest einer zweiten Form und zumindest einer dritten Form vor, wobei die erste Form eine freie Form, die zweite Form eine einfach glykosidische Form und die dritte Form eine mehrfach glykosidische Form ist. Insbesondere in diesem Fall beinhaltet die glykosidische Form die einfach glykosidische Form und die mehrfach glykosidische Form. Insbesondere kann die einfach glykosidische Form eine monosaccharidisch glykosidische Form sein. Hierdurch kann vorteilhaft eine hohe Bioverfügbarkeit erzielt werden.

Eine hohe Bioverfügbarkeit und/oder Wirksamkeit, insbesondere für Tiere mit kurzem und/oder insbesondere aviärem Verdauungstrakt und/oder für Tiere mit einem Verdauungstrakt mit eingeschränkter Aktivität von Mikroorganismen und/oder für Menschen und/oder Tiere mit beeinträchtigter Leber und/oder Nierenfunktion und/oder für Menschen und/oder Tiere, die Sonnenlicht und/oder UV-Licht nur in geringem Umfang ausgesetzt sind, wird erzielt, wenn ein Mengenverhältnis von Molekülen, die in der ersten oder der zweiten Form vorliegen (insbesondere folglich einer Summe einer Menge von Molekülen der ersten Form und von Molekülen der zweiten Form), und von Molekülen, die in der dritten Form vorliegen, wenigstens 2:1, insbesondere wenigstens 1:1, vorteilhaft wenigstens 3:2 beträgt. Insbesondere weisen dabei Moleküle der freien Form eine nochmals höhere Bioverfügbarkeit auf als Moleküle der zweiten Form, die wiederum vorteilhaft für Anwendungen geeignet sind, in denen eine gewisse zeitliche Verzögerung einer Wirkungsentfaltung gewünscht ist.

Ein seine Wirkung langsam entfaltendes Erzeugnis, welches insbesondere eine Einnahme in großen Zeitabständen erlaubt, vorteilhaft für Menschen und/oder für Tiere mit einem langen Verdauungstrakt und/oder einem Verdauungstrakt mit einer hohen Aktivität von Mikroorganismen, insbesondere einem multiplen Verdauungstrakt wie beispielsweise von Rindern, Schafen und/oder Ziegen, wird bereitgestellt, wenn das Mengenverhältnis von Molekülen, die in der ersten oder der zweiten Form vorliegen, und von Molekülen, die in der dritten Form vorliegen höchstens 1:2, insbesondere höchstens 1:1, vorteilhaft höchstens 2:3 beträgt. Vorzugsweise ist eine Wirkungsentfaltung des Erzeugnisses über das Mengenverhältnis zwischen freier beziehungsweise einfach glykosidischer Form und mehrfach glykosidischer Form anpassbar.

In einer nicht erfindungsgemäßen Ausführungsform wäre eine Verwendung des Erzeugnisses zur Behandlung und/oder zur Prävention von Erkrankungen im Zusammenhang mit Knochenmasse-Verlusten, Knochenfehlentwicklungen, Entzündungen, Infektionskrankheiten, neurodegenerativen Erkrankungen, Strahlenschäden, Diabetes, verminderter Fruchtbarkeit und/oder Hypercholesterinämie bei Tieren und/oder Menschen und/oder bei Sportlern und/oder bei körperlich schwer arbeitenden Menschen denkbar. Insbesondere eine Verwendung des, vorteilhaft als Tierfutter oder Tierfutterzusatz ausgebildeten, Erzeugnisses bei einer Tierzucht kann zu einem schnelleren und/oder gesünderen Wachstum von Zuchttieren, insbesondere Nutztieren, wie beispielsweise Schweinen und/oder Rindern und/oder vorzugsweise von Geflügel wie Hühnern und/oder Puten führen. Insbesondere bei Geflügel kann eine hohe Eierschalenqualität und/oder eine hohe Legeleistung erzielt und/oder eine Fleischqualität verbessert werden. Es ist aber auch denkbar, das Erzeugnis als, insbesondere überwiegend oder rein pflanzliches, Nahrungsergänzungsmittel für Menschen zu verwenden. Alternativ oder zusätzlich wäre denkbar, dass das Erzeugnis zur Behandlung und/oder zur Prävention von Erkrankungen im Zusammenhang mit Vitamin-D-Mangel bei Tieren und/oder Menschen verwendet wird. Ferner ist denkbar, dass das Erzeugnis zur Verbesserung einer Muskelentwicklung verwendet wird. Außerdem ist denkbar, dass das Erzeugnis zur Stärkung eines Immunsystems verwendet wird. Des Weiteren ist denkbar, dass das Erzeugnis zur Aufrechterhaltung einer Homöostase eines Kalzium- und Phosphathaushalts verwendet wird. Ferner ist denkbar, dass das Erzeugnis zu einer Verbesserung eines Prozesses eines Werfens, insbesondere bei Schweinen, und/oder zu einer zu einer Reduktion einer Wahrscheinlichkeit für Totgeburten, insbesondere bei Schweinen, verwendet wird. Eine Anwendung bei Sportlern ist ebenfalls denkbar.

Zudem wäre in einer nicht erfindungsgemäßen Ausführungsform eine Verwendung des Erzeugnisses, in dessen biologischem Material das Molekül in der ersten Form, in der zweiten Form und in der dritten Form vorliegt, zur Behandlung und/oder zur Prävention von Erkrankungen im Zusammenhang mit Vitamin-D-Mangel bei Tieren und/oder bei Menschen, insbesondere zur Behandlung und/oder zur Prävention von oben genannten Erkrankungen und/oder im Zusammenhang mit den oben genannten Zielen, denkbar. Vorteilhaft kann hierbei eine schnelle Wirkung und/oder eine hohe Bioverfügbarkeit insbesondere aufgrund der Moleküle der freien Form und der einfach glykosidischen Form erzielt werden. Ferner kann hierbei wahlweise eine nachhaltige und/oder zeitversetzte Wirkungsentfaltung insbesondere aufgrund der Moleküle der mehrfach glykosidischen Form erzielt werden. Insbesondere kann eine Bioverfügbarkeit und/oder eine Effizienz einer Hydrolyse in einem Verdauungstrakt in Abhängigkeit von einem Gehalt an Molekülen der freien Form, der einfach glykosidischen Form und der mehrfach glykosidischen Form gezielt und/oder anwendungsspezifisch angepasst werden. Insbesondere in dem Fall, dass das Mengenverhältnis von Molekülen, die in der ersten oder der zweiten Form vorliegen, und von Molekülen, die in der dritten Form vorliegen, wenigstens 1:1 beträgt, kann das Erzeugnis vorteilhaft zur Behandlung und/oder zur Prävention von Erkrankungen im Zusammenhang mit Vitamin-D-Mangel bei Menschen und/oder bei Tieren mit kurzem und/oder aviärem Verdauungstrakt und/oder mit einem Verdauungstrakt mit eingeschränkter Aktivität von Mikroorganismen und/oder Menschen oder Tieren mit reduzierter Leberfunktion und/oder reduzierter Nierenfunktion und/oder reduziertem Kontakt mit UV-Licht verwendet werden. Insbesondere in dem Fall, dass das Mengenverhältnis von Molekülen, die in der ersten oder der zweiten Form vorliegen, und von Molekülen, die in der dritten Form vorliegen, höchstens 1:1 beträgt, kann das Erzeugnis vorteilhaft zur Behandlung und/oder zur Prävention von Erkrankungen im Zusammenhang mit Vitamin-D-Mangel bei Menschen und/oder bei Tieren mit langem und/oder multiplem Verdauungstrakt und/oder mit einem Verdauungstrakt mit einer hohen Aktivität von Mikroorganismen verwendet werden.

Außerdem wäre in einer nicht durch die beanspruchte Erfindung abgedeckten Ausführungsform ein Verfahren zur Herstellung des Erzeugnisses, wobei das biologische Material aus einem biologischen, insbesondere pflanzlichen, Ausgangsstoff gewonnen wird, denkbar. Vorzugsweise ist der Ausgangsstoff als zumindest eine Pflanze und/oder zumindest ein Pilz und/oder zumindest ein Bakterium ausgebildet. Insbesondere kann der Ausgangsstoff zu einem Pulver und/oder einem Schnittgut und/oder einem Schreddergut verarbeitet werden. Dabei ist insbesondere denkbar, dass der Ausgangsstoff zu einem Pulver und/oder einem Schnittgut und/oder einem Schreddergut in unvergärter oder in zumindest teilweise vergärter Form verarbeitet wird. Insbesondere wird das biologische Material aus einer unkonzentrierten Form des Ausgangsstoffs gewonnen, vorzugsweise ohne eine Konzentration. Ferner ist denkbar, dass das biologische Material als ein Extrakt, insbesondere ein Trockenextrakt und/oder ein Flüssigextrakt, aus dem Ausgangsstoff und/oder einem aus dem Ausgangsstoff gewonnenen Zwischenprodukt gewonnen wird. Insbesondere enthält der Ausgangsstoff die Moleküle in einer Konzentration von wenigstens 1 $\mu$g/g, vorteilhaft von wenigstens 5 $\mu$g/g, besonders vorteilhaft von wenigstens 10 $\mu$g/g, vorzugsweise von wenigstens 50 $\mu$g/g und besonders bevorzugt von wenigstens 100 $\mu$g/g oder auch in einer höheren Konzentration wie beispielsweise 500 $\mu$g/g oder 1000 $\mu$g/g oder 2000 $\mu$g/g. Hierdurch kann vorteilhaft eine hohe Kosteneffizienz und/oder ein hoher Durchsatz erzielt werden. Ferner können hierdurch aufwändige Konzentrationsschritte vermieden werden.

In einer nicht erfindungsgemäßen Ausführungsform wäre denkbar, dass der Ausgangsstoff in einem Züchtungsverfahren gewonnen wird. Insbesondere könnte der Ausgangsstoff als eine Zielpflanze ausgebildet sein, die aus verschiedenen Pflanzen gezüchtet wird. Hierdurch kann vorteilhaft eine besonders ergiebige Quelle für Zielsubstanzen, insbesondere für freie Calictriol-Derivate und/oder Hydroxyzimtsäure-Derivate, geschaffen werden.

In einer weiteren nicht erfindungsgemäßen Ausführungsform wäre denkbar, dass eine Konzentration des Moleküls und/oder von einem Hydroxyzimtsäure-Derivat, insbesondere von dem Hydroxyzimtsäure-Derivat in verschiedenen Pflanzen, insbesondere der Gattung Cestrum, vorzugsweise der Art Cestrum diurnum, bestimmt wird, um für das Züchtungsverfahren geeignete Pflanzen auszuwählen, aus welchen insbesondere der Ausgangsstoff gewonnen werden kann und/oder welche vorteilhaft zur Schaffung einer weiteren Pflanze, beispielsweise mit einer höheren Konzentration des Hydroxyzimtsäure-Derivats, herangezogen werden kann. Die Konzentration wird dabei insbesondere wie oben beschrieben bestimmt. Insbesondere wird zumindest ein Screening im Hinblick auf einen hohen Gehalt des Moleküls, insbesondere in der freien Form, und/oder auf einen hohen Gehalt des Hydroxyzimtsäure-Derivats durchgeführt, um geeignete Pflanzen auszuwählen. Vorzugsweise können Eigenschaften von Pflanzen mit vorteilhaft hohem Gehalt des Moleküls, insbesondere in der freien Form, und/oder Eigenschaften von Pflanzen mit vorteilhaft hohem Gehalt des Hydroxyzimtsäure-Derivats kombiniert werden. Hierdurch kann insbesondere eine hohe Herstellungseffizienz erzielt und/oder ein Materialbedarf vorteilhaft reduziert werden.

In einer weiteren Ausführungsform, welche nicht durch die beanspruchte Erfindung abgedeckt ist, wäre denkbar, dass ein Mengenverhältnis von Molekülen der freien Form und der glykosidischen Form in verschiedenen Pflanzen, insbesondere der Gattung Cestrum, vorteilhaft der Art Cestrum diurnum bestimmt wird, um für das Züchtungsverfahren geeignete Pflanzen auszuwählen, aus welchen insbesondere der Ausgangsstoff gewonnen werden kann und/oder welche vorteilhaft zur Schaffung einer weiteren Pflanze, beispielsweise mit einem höheren Mengenverhältnis, herangezogen werden kann. Insbesondere wird das Mengenverhältnis wie oben beschrieben bestimmt. Vorteilhaft weist das Verfahren dabei zumindest einen Vorbehandlungsschritt auf, in welchem in zumindest einem Vorbehandlungsschritt eine Umwandlung von Molekülen der glykosidischen Form in Moleküle der freien Form durchgeführt wird. Besonders vorteilhaft wird die Umwandlung enzymatisch durchgeführt. Vorzugsweise wird ein Messgehalt an Molekülen der freien Form vor dem Vorbehandlungsschritt bestimmt. Besonders bevorzugt wird ein Referenzgehalt an Molekülen der freien Form nach dem Vorbehandlungsschritt bestimmt.

Ferner wäre in einer nicht erfindungsgemäßen Ausführungsform eine Verwendung des beschriebenen Verfahrens zur Bestimmung eines quantitativen Mengenverhältnisses von an einen Vitamin-D-Rezeptor bindenden Molekülen zur Schaffung eines Organismus, insbesondere einer Pflanze, mit zumindest einem biologischen Material, in dessen biologischem Material an einen Vitamin-D-Rezeptor bindende Moleküle, insbesondere Calcitriol-Derivate, in zumindest einer freien Form und in zumindest einer glykosidischen Form in einem bestimmten Mengenverhältnis vorliegen, wobei das Mengenverhältnis von Molekülen der freien Form und der glykosidischen Form bestimmt wird, denkbar.

Insbesondere kann diese Verwendung bei oben beschriebenem Verfahren zur Herstellung des Erzeugnisses angewandt werden. Es ist aber auch denkbar, dass gentechnische und/oder chemische Methoden zur Manipulation und/oder Veränderung eines existierenden Organismus wie beispielsweise einer Pflanze und/oder eines Bakterium zur Schaffung des Organismus verwendet werden. Vorteilhaft beträgt das Mengenverhältnis wenigstens 1:1, vorzugsweise wenigstens 3:2, bevorzugt wenigstens 7:3 und besonders bevorzugt wenigstens 4:1. Hierdurch kann vorteilhaft eine kostengünstige und/oder reichhaltige Quelle für Vitamin-D-artige Substanzen, insbesondere in einer freien Form, geschaffen werden.

Außerdem wäre in einer weiteren nicht erfindungsgemäßen Ausführungsform eine Verwendung des beschriebenen Verfahrens zur Bestimmung eines quantitativen Mengenverhältnisses von an einen Vitamin-D-Rezeptor bindenden Molekülen zur Schaffung eines Organismus, insbesondere einer Pflanze, welcher zumindest ein biologisches Material (10c) aufweist, in welchem an einen Vitamin-D-Rezeptor bindende Moleküle, insbesondere Calcitriol-Derivate, in der ersten Form, der zweiten Form und der dritten Form vorliegen, denkbar. Insbesondere könnte das Organismus mittels Züchtung geschaffen werden. Wie oben beschrieben könnte dabei je nach Anwendung der Organismus derart beschaffenen biologisches Material aufweisen, dass ein Mengenverhältnis von Molekülen, die in der ersten oder der zweiten Form vorliegen, und von Molekülen, die in der dritten Form vorliegen, wenigstens 1:2, insbesondere wenigstens 1:1, vorteilhaft wenigstens 3:2, besonders vorteilhaft wenigstens 7:3, vorzugsweise wenigstens 4:1 und besonders bevorzugt wenigstens 9:1 beträgt, und/oder derart beschaffenes biologisches Material aufweisen, dass ein Mengenverhältnis von Molekülen, die in der ersten oder der zweiten Form vorliegen, und von Molekülen, die in der dritten Form vorliegen, höchstens 2:1, vorteilhaft höchstens 1:1, besonders vorteilhaft höchstens 2:3, vorzugsweise 3:7, bevorzugt 1:4 und besonders bevorzugt 1:9 beträgt.

## Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:

Fig. 1    eine Identifizierung eines Organismus in einer schematischen Darstellung,
Fig. 2    ein Ablaufdiagramm eines Verfahrens zur Bestimmung eines quantitativen Mengenverhältnisses von an einen Vitamin-D-Rezeptor bindenden Molekülen,
Fig. 3    ein Diagramm mit beispielhaften Werten des Mengenverhältnisses in verschiedenen Pflanzen,
Fig. 4    ein beispielhaftes Absorptionsspektrum eines Calcitriol-Derivats,
Fig. 5    ein Diagramm mit beispielhaften Werten einer Aktivierung eines Vitamin-D-Rezeptors durch Calcitriol-Derivate verschiedener Proben,
Fig. 6    ein beispielhaftes Chromatogramm eines Extrakts mit biologischem Material des Organismus,
Fig. 7    ein Ablaufdiagramm eines Verfahrens zur Herstellung eines Erzeugnisses mit biologischem Material des Organismus,
Fig. 8    ein Erzeugnis mit biologischem Material des Organismus in einer schematischen Darstellung,
Fig. 9    einen weiteren Organismus in einer schematischen Darstellung,
Fig. 10    ein Nahrungsergänzungsmittel mit biologischem Material des weiteren Organismus in einer schematischen Darstellung,
Fig. 11    einen zweiten weiteren Organismus in einer schematischen Darstellung und
Fig. 12    ein weiteres Nahrungsergänzungsmittel mit biologischem Material des weiteren Organismus in einer schematischen Darstellung.

## Beschreibung der Ausführungsbeispiele

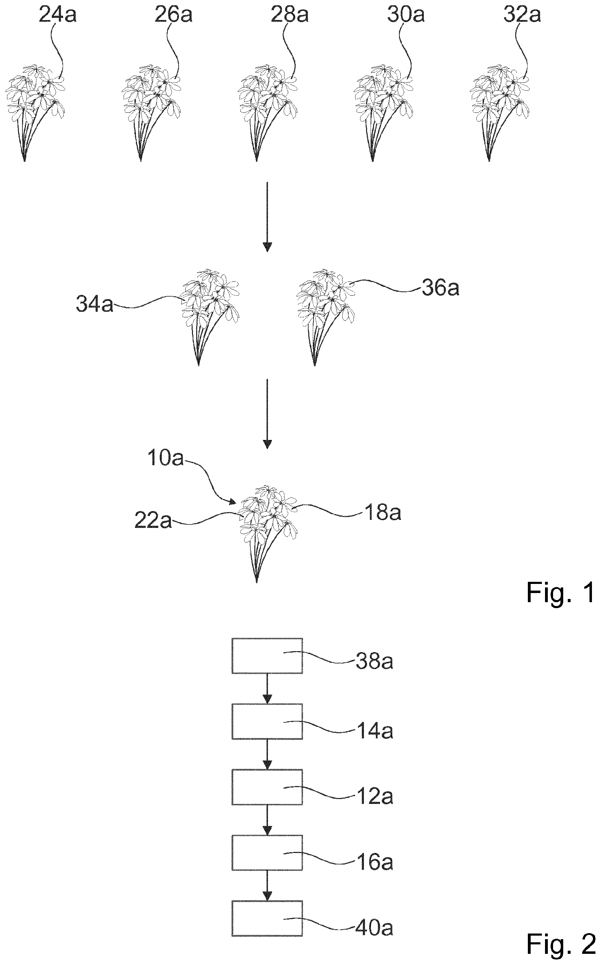

Die Figur 1 zeigt eine nicht erfindungsgemäße Ausführungsform einer Identifizierung eines Organismus 18a in einer schematischen Darstellung. Im vorliegenden Fall ist der Organismus 18a eine Pflanze der Gattung Cestrum. Ferner ist im vorliegenden Fall der Organismus 18a eine Pflanze der Art Cestrum diurnum. Es ist aber für die nachfolgende Beschreibung grundsätzlich auch denkbar, dass es sich bei dem Organismus um eine Pflanze einer anderen Art oder Gattung handelt. Der Organismus 18a weist einen Ausgangsstoff 22a auf. Im vorliegenden Fall handelt es sich bei dem Ausgangsstoff 22a um Pflanzenblätter. Es könnte sich alternativ oder zusätzlich aber auch um Wurzeln, Pflanzenstängel, Früchte und/oder andere Pflanzenteile handeln. Ferner ist denkbar, dass der gesamte Organismus den Ausgangsstoff bildet.

Der Ausgangsstoff 22a weist ein biologisches Material 10a auf. Im vorliegenden Fall weist das biologische Material 10a Zellbestandteile des Organismus 18a auf. Das biologische Material 10a weist an einen Vitamin-D-Rezeptor bindende Moleküle auf, die in zumindest einer freien Form und in zumindest einer glykosidischen Form in dem biologischen Material 10a vorliegen. Im vorliegenden Fall handelt es sich bei den Molekülen um Calcitriol-Derivate. Im vorliegenden Fall handelt es sich bei den Molekülen um Calcitriol. Insbesondere weist das biologische Material 10a Moleküle in einer Konzentration von mehr als 10 ppm auf. Dabei handelt es sich insbesondere um eine Gesamtkonzentration der Moleküle, d.h. um eine Summe einer Konzentration an Molekülen der freien Form und einer Konzentration an Molekülen der glykosidischen Form. Die Moleküle der freien Form und die Moleküle der glykosidischen Form liegen in einem bestimmten Mengenverhältnis in dem biologischen Material 10a vor. Im vorliegenden Fall beträgt das Mengenverhältnis etwa 6:1, d.h. es liegen etwa sechsmal so viele Moleküle der freien Form als Moleküle der glykosidischen Form in dem biologischen Material 10a vor. In analoger Weise kann das biologische Material zusätzlich oder alternativ Calcidiol und/oder Vitamin $D_3$ aufweisen.

Das biologische Material 10a weist zumindest ein von Vitamin $D_3$, Calcidiol und Calcitriol verschiedenes, an einen Vitamin-D-Rezeptor bindendes weiteres Molekül auf. Im vorliegenden Fall ist das weitere Molekül ein Calcitriol-Derivat. Das weitere Molekül liegt im vorliegenden Fall in einer freien Form und in einer glykosidischen Form in dem biologischen Material 10a vor. Das weitere Molekül liegt im vorliegenden Fall in einer Konzentration von wenigstens 5 ppm in dem biologischen Material vor. Es ist aber auch eine andere Konzentration denkbar, beispielsweise eine Konzentration von 2 ppm Dabei handelt es sich insbesondere um eine Gesamtkonzentration der weiteren Moleküle d.h. um eine Summe einer Konzentration an weiteren Molekülen der freien Form und einer Konzentration an weiteren Molekülen der glykosidischen Form.

Das biologische Material 10a weist zumindest ein Hydroxyzimtsäure-Derivat in einer Konzentration von wenigstens 100 µg/g auf. Eine Konzentration des Hydroxyzimtsäure-Derivats in dem biologischen Material 10a beträgt im vorliegenden Fall etwa 1500 µg/g. Dabei handelt es sich insbesondere um eine Gesamtkonzentration verschiedener Hydroxyzimtsäure-Derivate in dem biologischen Material. Bei dem Hydroxyzimtsäure-Derivat handelt es sich um 2-Hydroxyzimtsäure, 3-Hydroxyzimtsäure, 4-Hydroxyzimtsäure, 3,4,-Dihydroxyzimtsäure, Chlorogensäure, Cumarin, Ferulasäure oder Sinapinsäure.

Der Organismus 18a wird mittels Verwendung eines nachfolgend beschriebenen nicht erfindungsgemäßen Verfahrens zur Bestimmung eines quantitativen Mengenverhältnisses von an einen Vitamin-D-Rezeptor bindenden Molekülen (nachfolgend als "Bestimmungsverfahren" bezeichnet) identifiziert. Insbesondere wird der Organismus 18a mittels Verwendung des Bestimmungsverfahrens geschaffen, wobei ein Mengenverhältnis von Molekülen der freien Form und Molekülen der glykosidischen Form bestimmt wird. Der Organismus 18a wird in einem nicht erfindungsgemäßen Züchtungsverfahren aus geeigneten Organismen 34a, 36a gezüchtet. Die geeigneten Organismen 34a, 36a werden mittels Verwendung des Bestimmungsverfahrens identifiziert oder selbst aus entsprechend identifizierten geeigneten Organismen 24a, 28a aus einer Vielzahl von Organismen 24a, 26a, 28a, 30a, 32a gezüchtet. Im vorliegenden Fall handelt es sich bei den Organismen 24a, 26a, 28a, 30a, 32a, 34a, 36a um Pflanzen, insbesondere der Gattung Cestrum, vorteilhaft der Art Cestrum diurnum. Bei der nicht erfindungsgemäßen Züchtung wird insbesondere auch eine Konzentration des Moleküls und/oder des weiteren Moleküls und/oder des Hydroxyzimtsäure-Derivats in den Organismen 24a, 26a, 28a, 30a, 32a, 34a, 36a bestimmt, um für die Züchtung geeignete Organismen auszuwählen. Die dargestellten Organismen sowie deren Anzahl soll dabei lediglich als Beispiel verstanden werden. Es ist denkbar, dass eine weit größere Anzahl an Organismen als hier beschrieben untersucht und/oder identifiziert wird, beispielsweise eine Anzahl von 50 oder 100 oder 200 oder 500 oder 1000 oder 10000 oder noch mehr.

Die Figur 2 zeigt ein Ablaufdiagramm des nicht erfindungsgemäßen Bestimmungsverfahrens. Das Bestimmungsverfahren ist zur Bestimmung eines quantitativen Mengenverhältnisses von an einen Vitamin-D-Rezeptor bindenden Molekülen vorgesehen. Darunter, dass ein Verfahren zu einem Zweck vorgesehen ist, soll insbesondere verstanden werden, dass das Verfahren zumindest einen Verfahrensschritt beinhaltet, der speziell auf den Zweck abzielt und/oder dass das Verfahren gezielt auf den Zweck gerichtet ist und/oder dass das Verfahren einer Erfüllung des Zwecks dient und auf diese Erfüllung hin zumindest teilweise optimiert ist. Bei dem nicht erfindungsgemäßen Bestimmungsverfahren handelt es sich um ein Verfahren zur Bestimmung eines quantitativen Mengenverhältnisses von an einen Vitamin-D-Rezeptor bindenden Molekülen, die in zumindest einer freien Form und in zumindest einer glykosidischen Form in einem biologischen Material 10a vorliegen. Wie oben beschrieben handelt es sich bei den Molekülen vorteilhaft um Calcitriol-Derivate.

In einem ersten Verfahrensschritt 38a des nicht erfindungsgemäßen Bestimmungsverfahrens wird eine Probe mit biologischem Material entnommen, beispielsweise aus einem Organismus 18a, 24a, 26a, 28a, 30a, 32a, 34a, 36a. Beispielsweise werden Pflanzenblätter abgetrennt und kleingeschnitten.

Moleküle der freien Form werden in einem Messschritt 14a des nicht erfindungsgemäßen Bestimmungsverfahrens mittels eines lipophilen Lösungsmittels wie beispielsweise Methanol und/oder Ethanol und/oder Ethylacetat und/oder Dichlormethan und/oder Hexan und/oder THF aus wenigstens einem Teil der Probe extrahiert. Bei dem Teil der Probe handelt es sich um eine Messprobe. Eine Extraktion wird dabei durch ein Schütteln und/oder eine Behandlung in einem Ultraschallbad bei einer Temperatur zwischen 20°C und 50°C durchgeführt. Ein derart gewonnener Extrakt wird gefiltert. Anschließend wird das Lösungsmittel abgedampft. Ein derart gewonnener Trockenextrakt wird in einem geeigneten Lösungsmittelgemisch aufgenommen. Das geeignete Lösungsmittelgemisch ist im vorliegenden Fall an eine mobile Phase eines Hochleistungsflüssigkeitschromatographen angepasst. Mittels HPLC wird in dem Messschritt 14a ein Messgehalt an Molekülen der freien Form bestimmt. Der Messgehalt entspricht einem Gehalt an Molekülen der freien Form in der Messprobe.

In einem Vorbehandlungsschritt 12a des nicht erfindungsgemäßen Bestimmungsverfahrens werden Moleküle der glykosidischen Form mittels eines hydrophilen Lösungsmittels wie beispielsweise Wasser aus wenigstens einem Teil der Probe extrahiert. Der Vorbehandlungsschritt 12a wird insbesondere nach dem Messschritt 14a durchgeführt. Bei dem Teil der Probe handelt es sich um eine Referenzprobe. Zusätzlich wird dabei zumindest ein Antioxidant, beispielsweise Zitronensäure und/oder zumindest ein Gallat und/oder zumindest ein Sulfit und/oder Milchsäure und/oder Ascorbinsäure und/oder zumindest einem Ascorbat-Salz und/oder Zink und/oder Selen und/oder zumindest ein Diphosphat und/oder Zinnchlorid, zugegeben. Insbesondere wird ein Extrakt aus der Referenzprobe gewonnen, welcher lediglich Spuren von Molekülen der freien Form enthält. Ein Gehalt an Molekülen der glykosidischen Form in dem Extrakt entspricht vorteilhaft einem Gehalt an Molekülen der glykosidischen Form in der Referenzprobe. In dem Vorbehandlungsschritt 12a wird eine Umwandlung von Molekülen der glykosidischen Form in Moleküle der freien Form durchgeführt. Die Umwandlung wird insbesondere enzymatisch durchgeführt. Im vorliegenden Fall wird die Umwandlung mittels eines Enzymgemischs durchgeführt, welches zumindest zwei unterschiedliche Hydrolasen aufweist. Im vorliegenden Fall sind die verwendeten Hydrolasen pflanzlichen, mikrobiellen, tierischen oder fungalen Ursprungs, bevorzugt umfasst das Enzymgemisch zumindest eine Glucosidase und zumindest eine weitere Hydrolase, im vorliegenden Fall beispielsweise zumindest eine Galactosidase oder eine Amylase, wobei beispielsweise auch eine Pektinase oder eine Fructosidase denkbar sind. Wie oben erwähnt sind selbstverständlich auch weitere Hydrolasen denkbar. Darüber hinaus ist denkbar, dass das Enzymgemisch eine Mehrzahl unterschiedlicher Hydrolasen enthält, beispielsweise drei oder vier oder fünf unterschiedliche Hydrolasen. Das Enzymgemisch wird einem wie oben beschrieben gewonnenen Extrakt hinzugefügt. Die Umwandlung erfolgt bei einer Temperatur zwischen 20°C und 40°C. Insbesondere beträgt eine Effizienz der Umwandlung im vorliegenden Fall wenigstens 90 %. Ein Gesamtgehalt an Molekülen der freien Form in dem enzymatisch behandelten Extrakt nach der Umwandlung entspricht daher zumindest im Wesentlichen einem Gehalt an Molekülen der glykosidischen Form in der Referenzprobe vor der Umwandlung.

In einem Referenzbildungsschritt 16a des nicht erfindungsgemäßen Bestimmungsverfahrens wird ein Referenzgehalt an Molekülen der freien Form nach dem Vorbehandlungsschritt 12a bestimmt, insbesondere ein Referenzgehalt des während des Vorbehandlungsschritts 12a gewonnenen und enzymatisch behandelten Extrakts. Der Referenzgehalt wird analog zu dem Messgehalt bestimmt. Zu einer Bestimmung des Referenzgehalts werden Moleküle der freien Form analog zu dem Messschritt extrahiert. Die Bestimmung des Referenzgehalts erfolgt mittels HPLC. Der Referenzgehalt entspricht zumindest im Wesentlichen einem Gehalt an Molekülen der glykosidischen Form in der Referenzprobe, welche in dem Vorbehandlungsschritt wie oben erwähnt insbesondere in Moleküle der freien Form umgewandelt wurden.

In einem Auswertungsschritt 40a des nicht erfindungsgemäßen Bestimmungsverfahrens wird das Mengenverhältnis aus dem Messgehalt und dem Referenzgehalt bestimmt. Ein Gehalt an Molekülen der glykosidischen Form entspricht dabei dem Referenzgehalt. Das Mengenverhältnis kann berechnet werden als ein Quotient des Gehalts an Molekülen der freien Form und einem Gehalt an Molekülen der glykosidischen Form. Insbesondere kann das Mengenverhältnis berechnet werden als ein Quotient aus dem Messgehalt und dem Referenzgehalt.

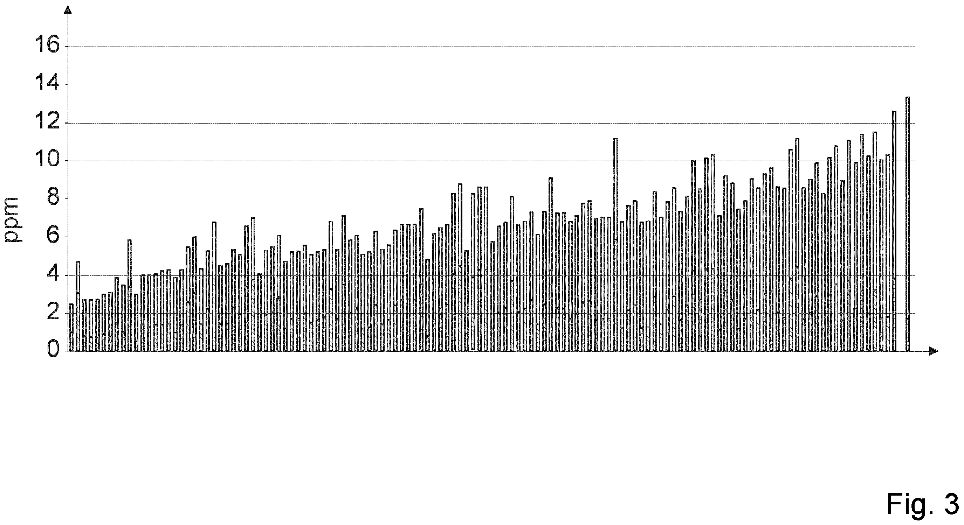

Wie erwähnt kann das nicht erfindungsgemäße Bestimmungsverfahren zur Identifizierung eines Organismus 18a verwendet werden, welcher zumindest ein biologisches Material 10a aufweist, in welchem an einen Vitamin-D-Rezeptor bindende Moleküle, insbesondere Calcitriol-Derivate, in zumindest einer freien Form und in zumindest einer glykosidischen Form vorliegen, wobei an einen Vitamin-D-Rezeptor bindende Moleküle nachgewiesen werden. Insbesondere wird ein Mengenverhältnis an Molekülen der freien Form und Molekülen der glykosidischen Form in dem biologischen Material 10a bestimmt. Auf diese Weise kann beispielsweise ein Screening an einer Vielzahl von Organismen durchgeführt werden, um Organismen mit einem vorteilhaft hohen Mengenverhältnis aufzuspüren. Ergebnisse eines derartigen Screenings sind beispielhaft in dem in der Figur 3 gezeigten Diagramm dargestellt. Im vorliegenden Fall wurde ein Mengenverhältnis von freiem Calcitriol (dargestellt als leere Balken) und glykosidischem Calcitriol (dargestellt als schraffierte Balken) in über 100 Pflanzen der Art Cestrum diurnum mittels des Bestimmungsverfahrens bestimmt. Für eine Züchtung wurden geeignete Pflanzen insbesondere hinsichtlich eines hohen Mengenverhältnisses ausgewählt. Ferner wurden Pflanzen mit einem hohen Gesamtgehalt von an einen Vitamin-D-Rezeptor bindenden Molekülen bevorzugt ausgewählt. Außerdem wurden Pflanzen mit einem hohen Gehalt an Hydroxyzimtsäure-Derivaten bevorzugt ausgewählt. Identifizierte Pflanzen wurden zur Züchtung herangezogen. Als Ergebnis der Züchtung wurde der Organismus 18a geschaffen (vgl. Figur 1), welcher einen hohen Gesamtgehalt an Calcitriol sowie ein hohes Mengenverhältnis aufweist (Messwert 42a). Im vorliegenden Fall beträgt der Gesamtgehalt etwa 14 ppm. Im vorliegenden Fall beträgt das Mengenverhältnis etwa 6:1. Es ist aber auch denkbar, einen Organismus mit einem höheren Gesamtgehalt, wie beispielsweise 20 ppm oder 30 ppm oder 40 ppm oder 50ppm oder 100 ppm und höher und/oder einem höheren Mengenverhältnis, wie beispielsweise 10:1 oder 20:1 oder 30:1 oder 40:1 zu züchten. Ein derart gezüchteter Organismus ist insbesondere als kostengünstige Quelle physiologisch leicht zugänglicher Vitamin-D-Derivate geeignet.

Wie oben erwähnt enthält der Organismus 18a ferner ein von Calcitriol, Calcidiol und Vitamin D₃ verschiedenes weiteres Calcitriol-Derivat. Ein Vorkommen eines solchen weiteren Calcitriol-Derivats in einer Pflanze der Gattung Cestrum, insbesondere der Art Cestrum diurnum, ist bislang nicht bekannt.

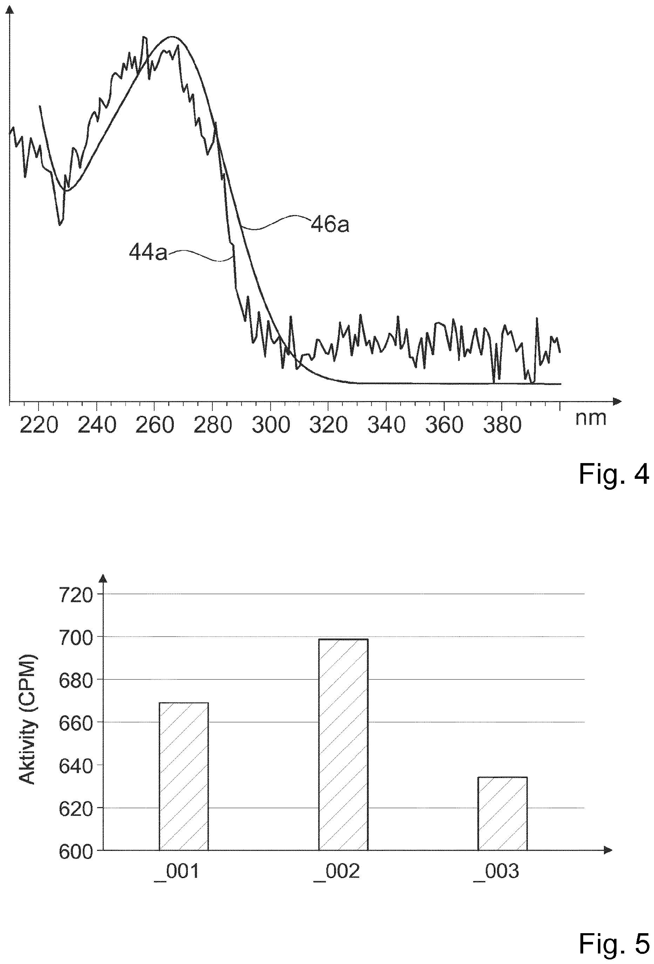

Die Figur 4 zeigt ein beispielhaftes Absorptionsspektrum 44a des weiteren Calcitriol-Derivats im Vergleich zu einem Absorptionsspektrum 46a von Calcitriol. Die Absorptionsspektren 44a, 46a zeigen einen Match Factor von 949. Insbesondere weisen die Absorptionsspektren 44a, 46a in einem Wellenlängenbereich unter 300 nm einen ähnlichen Verlauf auf. Es wird daher auf eine molekulare Ähnlichkeit des weiteren Calcitriol-Derivats zu Calcitriol geschlossen.

Die Figur 5 zeigt ein Diagramm mit beispielhaften Werten einer Aktivierung eines Vitamin-D-Rezeptors durch Calcitriol-Derivate verschiedener Proben. Die Aktivierung wurde in einem Bioassay bestimmt. Ein Gehalt an freiem Calcitriol, ein Gehalt an weiterem Calcitriol sowie ein Gesamtgehalt an Calcitriol und dem Calcitriol-Derivat sind in der nachfolgenden Tabelle angeführt.

| Probe | Freies Calcitriol (ng/ml) | Weiteres Calcitriol-Derivat (ng/ml) | Gesamtgehalt (ng/ml) |
|---|---|---|---|
| 001 | 3100 | 1466 | 4566 |
| 002 | 2620 | 2960 | 5580 |
| 003 | 180 | 1700 | 1880 |

Die Probe 001 stammt dabei von dem Organismus 18a, während die Probe 002 und die Probe 003 von anderen Pflanzen der Gattung Cestrum diurnum stammen. Die Probe 003 enthält insbesondere eine vergleichsweise geringe Menge an freiem Calcitriol, zeigt jedoch eine Aktivierung des Vitamin-D-Rezeptors. Die Probe 002 zeigt eine höhere Aktivierung als die Probe 001, obwohl ein Gehalt an freiem Calcitriol in der Probe 001 höher ist als in der Probe 002. Hierdurch wird insbesondere der Schluss nahegelegt, dass das weitere Calcitriol-Derivat eine Aktivierung eines Vitamin-D-Rezeptors hervorrufen kann. Insbesondere ist zu erwarten, dass das weitere Calcitriol-Derivat eine ähnliche physiologische Wirkung hat wie freies Calcitriol.

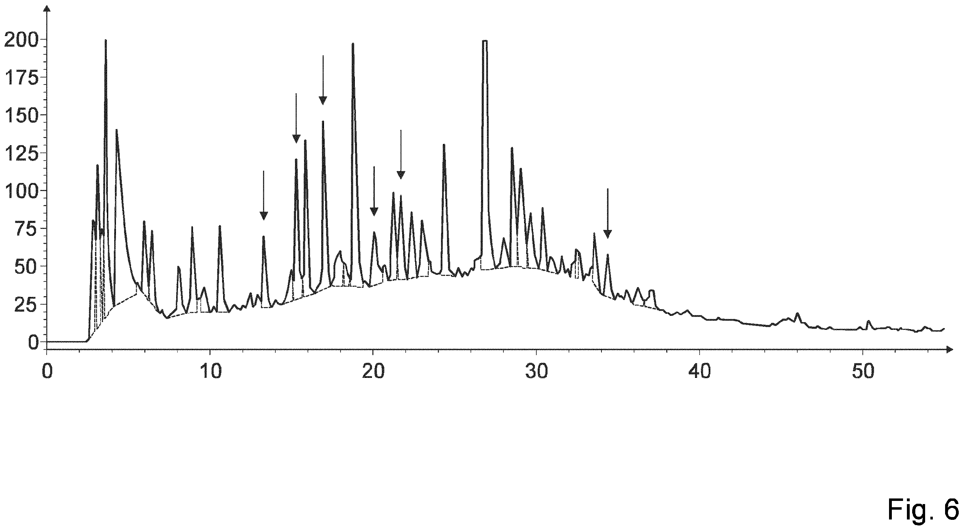

Die Figur 6 zeigt ein beispielhaftes Chromatogramm eines Extrakts mit biologischem Material 10a des Organismus 18a. Peaks, die Hydroxyzimtsäure-Derivaten zugeschrieben werden können, sind mit Pfeilen markiert. Wie oben erwähnt beträgt ein mittels HPLC bestimmter Gehalt an Hydroxyzimtsäure-Derivaten in dem biologischen Material 10a über 1500 μg/g.

Biologisches Material 10a des Organismus 18a eignet sich aufgrund eines hohen Mengenverhältnisses an Molekülen der freien Form und Molekülen der glykosidischen Form und/oder aufgrund eines hohen Gehalts an zumindest einem weiteren Calcitriol-Derivat und/oder aufgrund eines hohen Gehalts an Hydroxyzimtsäure zur Behandlung und/oder zur Prävention von Erkrankungen im Zusammenhang mit Knochenmasse-Verlusten, Knochenfehlentwicklungen, Entzündungen, Infektionskrankheiten, neurodegenerativen Erkrankungen, Strahlenschäden, Diabetes, verminderter Fruchtbarkeit und/oder Hypercholesterinämie bei Tieren und/oder Menschen und/oder bei Sportlern und/oder bei körperlich schwer arbeitenden Menschen. Ebenso ist eine Anwendung bei Sportlern und/oder körperlich schwer arbeitenden Menschen denkbar. Wie oben beschrieben kann ein Organismus mit einem biologischen Material mit einem vorteilhaften und/oder gewünschten Mengenverhältnis freier und glykosidischer Moleküle und/oder mit einem Gehalt an Vitamin-D-artigen Substanzen und/oder Hydroxyzimtsäure-Derivaten geschaffen werden.

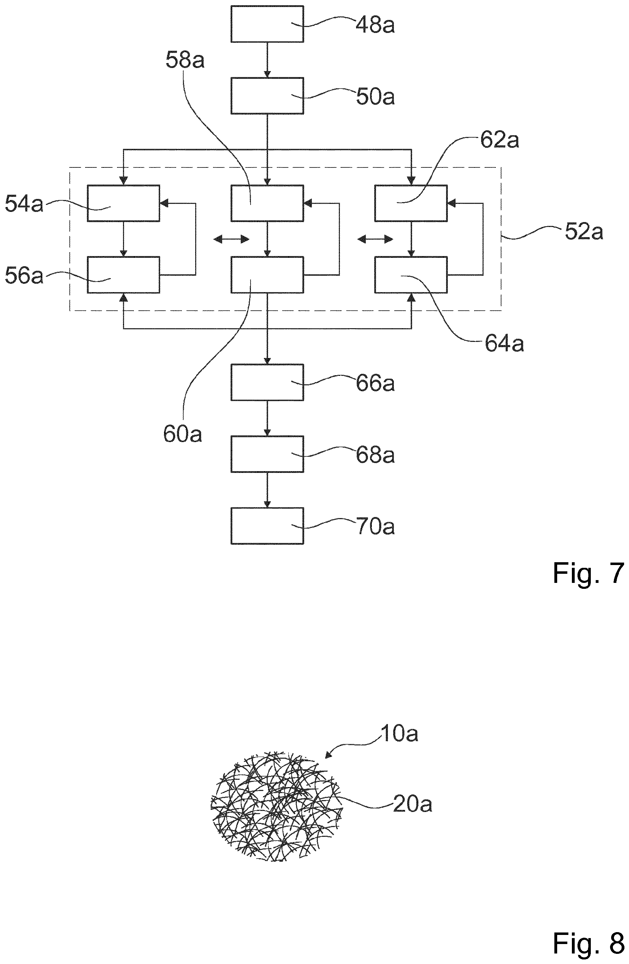

Die Figur 7 zeigt ein Ablaufdiagramm eines nicht erfindungsgemäßen Verfahrens zur Herstellung eines Erzeugnisses 20a zur Zufuhr zumindest eines an einen Vitamin-D-Rezeptor bindenden Moleküls, welches das biologische Material 10a aufweist (vergleiche Figur 8). In einem ersten Verfahrensschritt 48a werden geeignete Organismen ausgewählt, die für ein Screening wie oben beschrieben infrage kommen. In einem zweiten Verfahrensschritt 50a wird das Screening durchgeführt. Insbesondere wird dabei biologisches Material jedes ausgewählten Organismus entnommen. Geeignete Organismen werden anschließend in einem Züchtungsverfahren 52a verwendet, um einen Organismus mit gewünschten Eigenschaften zu schaffen. In einem Verfahrensschritt 54a wird ein Gehalt von an einen Vitamin-D-Rezeptor bindenden Molekülen in geeigneten Organismen bestimmt und diese in einem Verfahrensschritt 56a zur Züchtung von Organismen mit einem höheren Gehalt verwendet. Alternativ oder zusätzlich wird in einem Verfahrensschritt 58a ein Mengenverhältnis von Molekülen der freien Form und Molekülen der glykosidischen Form in geeigneten Organismen mittels des Bestimmungsverfahrens bestimmt und diese in einem Verfahrensschritt 60a zur Züchtung von Organismen mit einem höheren Mengenverhältnis verwendet. Alternativ oder zusätzlich wird in einem Verfahrensschritt 62a ein Gehalt an Hydroxyzimtsäure-Derivaten in geeigneten Organismen bestimmt und diese in einem Verfahrensschritt 64a zur Züchtung von Organismen mit einem höheren Gehalt an Hydroxyzimtsäure-Derivaten verwendet. Insbesondere können die Verfahrensschritte 54a, 56a, 58a, 60a, 62a, 64a iterativ und/oder parallel und/oder wechselweise durchgeführt werden. Beispielsweise kann zunächst ein Gehalt an Calcitriol-Derivaten optimiert und anschließend ein Mengenverhältnis von freien Calcitriol-Derivaten und glykosidischen Calcitriol-Derivaten und/oder ein Gehalt an Hydroxyzimtsäure-Derivaten maximiert werden. Ebenso sind andere Kombination und Reihenfolgen einer derartigen Optimierung denkbar.

In einem Verfahrensschritt 66a des nicht erfindungsgemäßen Verfahrens wird ein in dem nicht erfindungsgemäßen Züchtungsverfahren 52a geschaffener Organismus kultiviert und/oder angebaut. Beispielsweise werden geeignete Pflanzen angebaut und entsprechend vermehrt. Es ist auch denkbar, dass verschiedene Organismen mit verschiedenem biologischem Material aus dem Züchtungsverfahren 52a hervorgehen, die anschließend in Kombination kultiviert und/oder angebaut und/oder verwendet werden. In einem Verfahrensschritt 68a werden die kultivierten und/oder angebauten Organismen verarbeitet, beispielsweise geerntet. Aus den Organismen wird ein Ausgangsstoff gewonnen. Geeignetes biologisches Material wird aus dem Ausgangsstoff gewonnen. Im vorliegenden Fall wird der Organismus 18a angebaut und geerntet. Als Ausgangsstoff 22a (vgl. Figur 1) werden Pflanzenblätter des Organismus entfernt. In einem Verfahrensschritt 70a wird das Erzeugnis 20a hergestellt, insbesondere aus dem Ausgangsstoff. Im vorliegenden Fall wird aus dem Ausgangsstoff 22a das biologische Material 10a gewonnen. Das biologische Material 10a kann beispielsweise in Form eines Flüssigextrakts oder in Form eines Trockenextrakts oder als Schnittgut gewonnen werden. Bei dem Erzeugnis kann es sich beispielsweise um ein Nahrungsergänzungsmittel, ein Tierfutter oder einen Tierfutterzusatz handeln.

Die Figur 8 zeigt das nicht erfindungsgemäße Erzeugnis 20a mit biologischem Material 10a des Organismus 18a in einer schematischen Darstellung. Im vorliegenden Fall ist das Erzeugnis 20a ein Schnittgut aus Pflanzenblättern des Organismus 18a. Das Erzeugnis 20a ist im vorliegenden Fall ein Tierfutterzusatz. Insbesondere ist das Erzeugnis 20a als Quelle für Vitamin-D-artige Substanzen sowie für Hydroxyzimtsäure geeignet. Vorteilhaft kann das Erzeugnis 20a einem Tierfutter beigemischt und bei einer Tierhaltung verwendet werden. Dadurch können die oben beschriebenen vorteilhaften Effekte erzielt werden. Das Erzeugnis kann zusätzlich und/oder alternativ zur Behandlung und/oder zur Prävention von Erkrankungen im Zusammenhang mit Knochenmasse-Verlusten, Knochenfehlentwicklungen, Entzündungen, Infektionskrankheiten, neurodegenerativen Erkrankungen, Strahlenschäden, Diabetes, verminderter Fruchtbarkeit und/oder Hypercholesterinämie bei Tieren und/oder Menschen verwendet werden. Auch ein Einsatz bei Sportlern und/oder bei körperlich schwer arbeitenden Menschen ist denkbar. Es ist aber auch denkbar, einen Organismus zu schaffen, der zusätzlich im Hinblick auf einen Nährwert optimiert wird. Ein Erzeugnis analog zu dem Erzeugnis 20a kann dann beispielsweise direkt als Tierfutter verwendet werden.

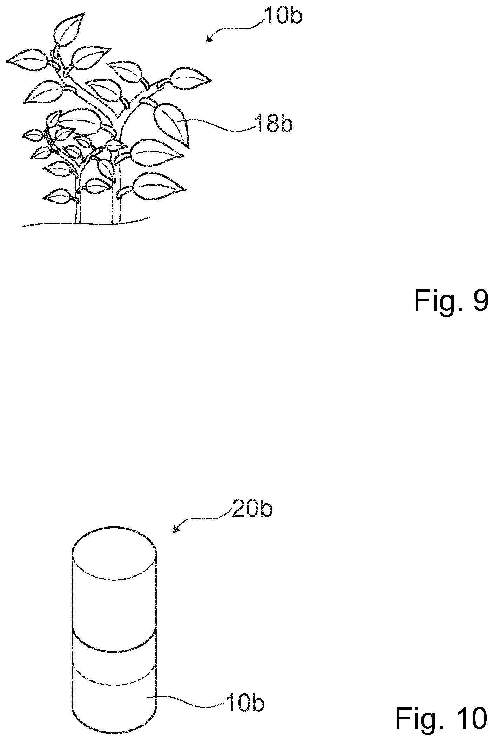

In den Figuren 9 bis 12 sind zwei weitere Ausführungsbeispiele gezeigt. Die nachfolgenden Beschreibungen beschränken sich im Wesentlichen auf die Unterschiede zwischen den Ausführungsbeispielen, wobei bezüglich gleich bleibender Bestandteile, Merkmale und Funktionen auf die Beschreibung des Ausführungsbeispiels der Figuren 1 bis 8 verwiesen werden kann. Zur Unterscheidung der Ausführungsbeispiele ist der Buchstabe a in den Bezugszeichen des Ausführungsbeispiels in den Figuren 1 bis 8 durch die Buchstaben b und c in den Bezugszeichen der Ausführungsbeispiele der Figuren 9 bis 12 ersetzt. Bezüglich gleich bezeichneter Bestandteile, insbesondere in Bezug auf Bestandteile mit gleichen Bezugszeichen, kann grundsätzlich auch auf die Zeichnungen und/oder die Beschreibung des Ausführungsbeispiels der Figuren 1 bis 8 verwiesen werden.

Die Figur 9 zeigt einen weiteren Organismus 18b in einer schematischen Darstellung. Der weitere Organismus 18b wurde analog zu dem Organismus 18a des nicht erfindungsgemäßen Beispiels der Figuren 1 bis 8 gezüchtet. Der weitere Organismus 18b weist biologisches Material 10b mit einem höheren Gehalt an Hydroxyzimtsäure-Derivaten auf als der Organismus 18a. Der Organismus 18b wurde im Hinblick auf einen hohen Gehalt an Hydorxyzimtsäure-Derivaten optimiert. Im vorliegenden Fall ist der weitere Organismus 18b eine Pflanze der Gattung Cestrum. Beispielsweise kann der weitere Organismus 18b eine Pflanze der Art Cestrum nocturnum oder Cestrum parqui sein. Ferner ist denkbar, dass der weitere Organismus 18b eine Pflanze einer beliebigen anderen Art der Gattung Cestrum ist wie beispielsweise Cestrum nocturnum x, Cestrum diurnum oder dergleichen.

Die Figur 10 zeigt ein weiteres Erzeugnis 20b mit biologischem Material 10b des weiteren Organismus 18b. Das Erzeugnis 20b ist als ein Nahrungsergänzungsmittel ausgebildet. Im vorliegenden Fall ist das Erzeugnis 20b als eine Kapsel ausgebildet, welche das biologische Material 10b in Form eines Pulvers enthält. Wie oben erwähnt sind aber auch andere Arten von Erzeugnissen wie beispielsweise Nahrungsmittel, Pharmazeutika, Kosmetika, etc. denkbar.

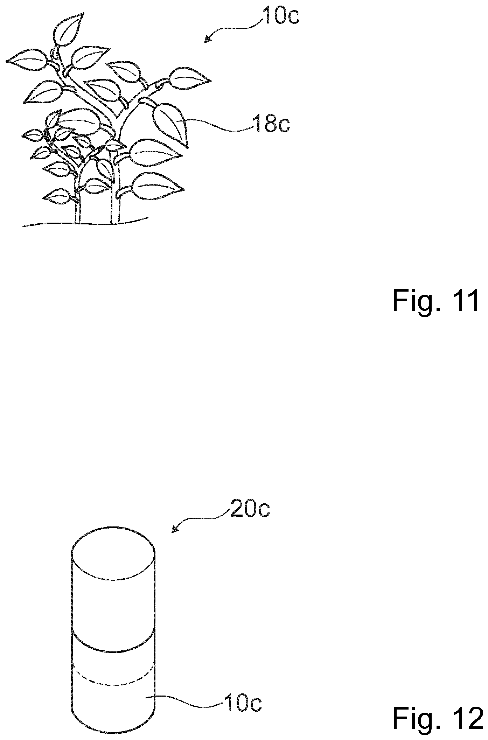

Die Figur 11 zeigt einen zweiten weiteren Organismus 18c in einer schematischen Darstellung. Der zweite weitere Organismus 18c weist ein biologisches Material 10c auf. Das biologische Material 10c weist zumindest ein an einen Vitamin-D-Rezeptor bindendes Molekül, insbesondere zumindest ein Calcitriol-Derivat, auf.

Die Figur 12 zeigt ein zweites weiteres Erzeugnis 20c mit biologischem Material 10c des Organismus. Das Erzeugnis 20c ist als ein Nahrungsergänzungsmittel oder als ein Futtermittelzusatz oder dergleichen ausgebildet. Beispielsweise enthält das Erzeugnis 20c das biologische Material 10c in Form eines Pulvers, wobei wie oben erwähnt auch denkbar ist, dass das Erzeugnis 20c ein Schnittgut ist.

Das Molekül liegt in dem biologischen Material 10c in zumindest einer ersten Form, zumindest einer zweiten Form und zumindest einer dritten Form vor, wobei die erste Form eine freie Form, die zweite Form eine einfach glykosidische Form und die dritte Form eine mehrfach glykosidische Form ist. Im vorliegenden Fall beträgt ein Mengenverhältnis von Molekülen, die in der ersten oder der zweiten Form vorliegen, und von Molekülen, die in der dritten Form vorliegen, wenigstens 1:2, vorteilhaft wenigstens 1:1 und besonders vorteilhaft wenigstens 3:2. Insbesondere weist das biologische Material im vorliegenden Fall eine hohe Konzentration von Molekülen der ersten und der zweiten Form im Vergleich zu einer Konzentration von Molekülen der dritten Form auf. Insbesondere ist, wie oben beschrieben, das Erzeugnis zur Behandlung und/oder zur Prävention von Erkrankungen im Zusammenhang mit Vitamin-D-Mangel bei Tieren und/oder bei Menschen verwendbar, insbesondere, falls eine hohe Bioverfügbarkeit angestrebt werden soll, beispielsweise im Fall von Tieren mit aviären Verdauungssystemen und/oder im Fall von Menschen und/oder Tieren mit eingeschränkter Leber- und/oder Nierenfunktion und/oder mit reduziertem Kontakt mit Sonnenlicht. Es ist auch denkbar, dass das Mengenverhältnis höchstens 2:1, vorteilhaft höchstens 1:1 und besonders vorteilhaft höchstens 2:3 beträgt. Insbesondere ist denkbar, dass das Erzeugnis 20c Moleküle der dritten Form in einer vergleichsweise hohen Konzentration aufweist, um beispielsweise eine langsame Abgabe aufgrund einer Abspaltung von Zucker-Resten erst im Verdauungstrakt zu ermöglichen. Insbesondere ist ein entsprechendes Erzeugnis 20c zur Behandlung und/oder Prävention von Erkrankungen im Zusammenhang mit Vitamin-D-Mangel bei Menschen und/oder bei Tieren verwendbar, insbesondere bei Tieren mit langem Verdauungstrakt wie beispielsweise bei Rindern, Schafen oder Ziegen.

Das oben beschriebene nicht erfindungsgemäße Verfahren zur Bestimmung eines quantitativen Mengenverhältnisses von an einen Vitamin-D-Rezeptor bindenden Molekülen kann in diesem Zusammenhang einen zusätzlichen Extraktionsschritt umfassen, in welchem zumindest ein Teil der Moleküle der glykosidischen Form mittels zumindest eines Extraktionsmittels, insbesondere eines Lösungsmittels und/oder eines Lösungsmittelgemischs, aus dem biologischen Material 10c extrahiert wird, das weniger polar ist als Wasser. Beispielsweise kann das Extraktionsmittel ein Gemisch von Wasser und Ethanol oder Methanol oder einem anderen geeigneten Lösungsmittel sein, wie insbesondere oben beschrieben. Ferner sind auch Extraktionsmittel denkbar, die eine Mehrzahl unterschiedlicher Lösungsmittel, beispielsweise drei oder vier oder noch mehr, enthalten. Ebenso sind Extraktionsmittel bekannt, die lediglich ein einzelnes Lösungsmittel umfassen. Vorzugsweise werden in dem Extraktionsschritt die Moleküle der zweiten Form extrahiert. Bei den Molekülen der zweiten Form handelt es sich ebenfalls um Moleküle der glykosidischen Form. Die Moleküle der zweiten Form werden nach deren Extraktion analog zu dem Referenzbildungsschritt in Moleküle der freien Form umgewandelt. Eine Konzentration der Moleküle der zweiten Form wird entsprechend nach deren Umwandlung in Moleküle der zweiten Form wie oben beschrieben bestimmt. Vorteilhaft können demnach in dem biologischen Material enthaltene Mengen an Molekülen der ersten Form, der zweiten Form und der dritten Form sowie entsprechende Mengenverhältnisse bestimmt werden. Vorzugsweise werden zunächst die Moleküle der dritten Form mit Wasser als Lösungsmittel aus dem biologischen Material 10c extrahiert, ehe die Moleküle der zweiten Form mittels des Extraktionsmittels aus dem biologischen Material 10c extrahiert werden. Es ist aber auch denkbar, unterschiedliche Messproben für die beiden Extraktionen zu verwenden.

Das derart angepasste nicht erfindungsgemäße Verfahren kann zur Identifizierung eines Organismus 18c, insbesondere einer Pflanze, welcher zumindest ein biologisches Material 10c aufweist, in welchem an einen Vitamin-D-Rezeptor bindende Moleküle, insbesondere Calcitriol-Derivate, in zumindest einer ersten Form, zumindest einer zweiten Form und zumindest einer dritten Form vorliegen, wobei die erste Form eine freie Form, die zweite Form eine einfach glykosidische Form und die dritte Form eine mehrfach glykosidische Form ist, verwendet werden. Ferner kann ein Erzeugnis 20c mit einem entsprechend identifizierten Organismus bereitgestellt werden.

Darüber hinaus kann das derart angepasste nicht erfindungsgemäße Verfahren verwendet werden, um den entsprechenden Organismus 18c insbesondere mittels Züchtung zu schaffen. Insbesondere können Organismen einer bestehenden Generation dabei wahlweise im Hinblick auf einen hohen Gehalt von Molekülen der ersten Form und/oder der zweiten Form und/oder der dritten Form ausgewählt werden, wobei geeignete Organismen zur kostengünstigen Herstellung von unterschiedlichen Erzeugnissen beispielsweise für die oben erwähnten Anwendungen geschaffen werden können. Beispielsweise kann ein Mengenverhältnis von Molekülen, die in der ersten oder der zweiten Form vorliegen, und von Molekülen, die in der dritten Form vorliegen, wenigstens 1:1, vorteilhaft wenigstens 3:2 betragen. Es ist aber auch denkbar, dass ein Mengenverhältnis von Molekülen, die in der ersten oder der zweiten Form vorliegen, und von Molekülen, die in der dritten Form vorliegen, höchstens 2:1, vorteilhaft höchstens 1:1, besonders vorteilhaft höchstens 2:3 beträgt. Selbstverständlich ist im Fall der Ausführungsbeispiele der Figuren 1 bis 10 in analoger Weise denkbar, dass zumindest einige der Moleküle der glykosidischen Form jeweils entweder Moleküle der zweiten Form oder Moleküle der dritten Form sind.

**Patentansprüche**

1. Erzeugnis, insbesondere Nahrungsmittelzusatz, Futtermittel, Nahrungsmittel, Pharmazeutikum, Kosmetikum und/oder Tierfutterzusatz, zur Zufuhr zumindest eines an einen Vitamin-D-Rezeptor bindenden Moleküls, und zwar Vitamin $D_3$ und/oder Calcitriol und/oder Calcidiol, mit zumindest einem biologischen Material (10a; 10b; 10c), welches das Molekül aufweist und bei welchem es sich um Pflanzen und/oder Pflanzenteile und/oder zumindest einen daraus gewonnenen Extrakt und/oder eine daraus hergestellte Lösung und/oder Dispersion handelt, **dadurch gekenn-**

**zeichnet, dass** das Molekül in dem biologischen Material (10c) in zumindest einer ersten Form, zumindest einer zweiten Form und zumindest einer dritten Form vorliegt, wobei die erste Form eine freie Form, die zweite Form eine einfach glykosidische Form und die dritte Form eine mehrfach glykosidische Form ist, wobei ein Mengenverhältnis von Molekülen, die in der ersten oder der zweiten Form vorliegen, und von Molekülen, die in der dritten Form vorliegen, wenigstens 1:2 und höchstens 2:1 beträgt und das biologische Material die Moleküle in einer Konzentration von wenigstens 50 μg/g enthält, wobei das biologische Material (10a; 10b) zumindest ein Hydroxyzimtsäure-Derivat in einer Konzentration von wenigstens 100 μg/g enthält, wobei es sich bei dem Hydroxyzimtsäure-Derivat um 2-Hydroxyzimtsäure, 3-Hydroxyzimtsäure, 4-Hydroxyzimtsäure, 3,4,-Dihydroxyzimtsäure, Chlorogensäure, Cumarin, Ferulasäure oder Sinapinsäure handelt.

2. Erzeugnis nach Anspruch 1, **dadurch gekennzeichnet, dass** das Molekül in dem biologischen Material (10a; 10b) in zumindest einer freien Form und in zumindest einer glykosidischen Form vorliegt, wobei ein Mengenverhältnis von Molekülen der freien Form und Molekülen der glykosidischen Form wenigstens 1:1 beträgt.

3. Erzeugnis nach einem der vorhergehenden Ansprüche, **dadurch gekennzeichnet, dass** das biologische Material (10a; 10b) von zumindest einer Pflanze der Gattung Cestrum stammt.

4. Erzeugnis nach Anspruch 3, **dadurch gekennzeichnet, dass** die Pflanze von der Art Cestrum diurnum ist.

**Claims**

1. Product, in particular nutritional supplement, animal feed, nutritional product, pharmaceutical product, cosmetic product and/or animal feed supplement, for supplying at least one molecule that binds to a vitamine D receptor, namely vitamine $D_3$ and/or calcitriol and/or calcidiol, with at least one biologic material (10a; 10b; 10c) that comprises the molecule and is realized as plants and/or parts of plants and/or an extract obtained therefrom, and/or a solution and/or dispersion produced therefrom,

characterised in that the molecule is present in the biological material (10c) in at least one first form, at least one second form and at least one third form,
the first form being a free form, the second form being a simply glycosidic form and the third form being a multiply glycosidic form,
wherein a quantitative proportion of molecules present in the first or second form and of molecules present in the third form is at least 1:2 and maximally 2:1,
and the biologic material contains the molecules in a concentration of at least 50 μg/g,
wherein the biological material (10a; 10b) contains at least one hydroxycinnamic acid derivate in a concentration of at least 100 μg/g, the hydroxycinnamic acid derivate being 2-hydroxy cinnamic acid, 3-hydroxy cinnamic acid, 4-hydroxy cinnamic acid, 3,4-dihydroxy cinnamic acid, chlorogenic acid, cumarine, ferulic acid or sinapic acid.

2. Product according to claim 1,

characterised in that the molecule is present in the biologic material (10a; 10b) in at least one free form and in at least one glycosidic form,
wherein a quantitative proportion of molecules in the free form and molecules in the glycosidic form is at least 1:1.

3. Product according to one of the preceding claims,
characterised in that the biologic material (10a; 10b) origins from at least one plant of the genus Cestrum.

4. Product according to claim 3,
characterised in that the plant belongs to the species Cestrum diurnum.

**Revendications**

1. Produit, en particulier additif de produit alimentaire, aliment pour animaux, produit alimentaire, produit pharmaceutique, produit cosmétique et/ou additif d'aliment pour animaux, pour un apport d'au moins un molécule se liant à un récepteur de la vitamine D, notamment la vitamine $D_3$ et/ou calcitriol et/ou calcidiol,

avec au moins un matériau biologique (10a ; 10b ; 10c) comprenant le molécule et réalisé comme des plantes et/ou des parties de plantes et/ou au moins un extrait obtenu des plantes et/ou des parties de plantes et/ou une solution et/ou dispersion produite des plantes et/ou des parties de plantes,
**caractérisé en ce que** le molécule est présent dans le matériau biologique (10c) en au moins une première forme, au moins une deuxième forme et au moins une troisième forme, la première forme étant une forme libre, la deuxième forme étant une forme simplement glycosidique et la troisième forme étant une forme multiplement glycosidique,
où un rapport quantitatif de molécules présents en la première ou la deuxième forme et de molécules présents en la troisième forme est au moins 1:2 et maximale-ment 2:1,
et le matériau biologique contient les molécules dans une concentration d'au moins 50 $\mu$g/g,
où le matériau biologique contient au moins un dérivé de l'acide cinnamique dans une concentration d'au moins 100 $\mu$g/g, le dérivé de l'acide cinnamique étant l'acide 2-hydroxy-cinnamique, l'acide 3-hydroxy-cinnamique, l'acide 4-hydroxy-cinnamique, l'acide 3,4-dihydroxy-cinnamique, l'acide chlorogénique, la coumarine, l'acide féru-lique ou l'acide sinapique.

2.  Produit selon la revendication 1,
    **caractérisé en ce que** le molécule est présent dans le matériau biologique (10a ; 10b) en au moins une forme libre et en au moins une forme glycosidique, le rapport quantitatif de molécules en forme libre et de molécules en forme glycosidique étant au moins 1:1.

3.  Produit selon l'une des revendications précédentes,
    **caractérisé en ce que** le matériau biologique (10a ; 10b) s'origine d'au moins une plante du genre Cestrum.

4.  Produit selon la revendication 3,
    **caractérisé en ce que** la plante est de l'espèce Cestrum diurnum.

24a    26a    28a    30a    32a

34a    36a

10a    18a
22a

Fig. 1

38a

14a

12a

16a

40a

Fig. 2

Fig. 3

Fig. 4

Fig. 5

EP 3 432 890 B1

Fig. 6

48a

58a

50a

62a

54a

52a

56a

64a

60a

66a

68a

70a

Fig. 7

10a

20a

Fig. 8

10b

18b

Fig. 9

20b

10b

Fig. 10

10c

18c

Fig. 11

20c

10c

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007066355 A1

**In der Beschreibung aufgeführte Nicht-Patentliteratur**

- **BEGUM, A. S. ; GOYAL, M.** Phcog Mag - Review Article Research and Medicinal potential of the genus Cestrum (Solanaceae)- A review. *Pharmacognosy Reviews,* 2007, vol. 1 (2), 320-332